(19) Europäisches Patentamt
European Patent Office
Office européen des brevets

(11) EP 1 009 750 B1

(12) **FASCICULE DE BREVET EUROPEEN**

(45) Date de publication et mention de la délivrance du brevet:
**31.07.2002 Bulletin 2002/31**

(21) Numéro de dépôt: **97911309.9**

(22) Date de dépôt: **21.10.1997**

(51) Int Cl.[7]: **C07F 9/30**, A61K 31/66, C07F 9/32

(86) Numéro de dépôt international:
**PCT/FR97/01884**

(87) Numéro de publication internationale:
**WO 98/18803 (07.05.1998 Gazette 1998/18)**

(54) **NOUVEAUX DERIVES D'(ALPHA-AMINOPHOSPHINO)PEPTIDES, LEUR PROCEDE DE PREPARATION ET LEURS APPLICATIONS THERAPEUTIQUES**

NEUE (ALPHA-AMINOPHOSPHINO) PEPTIDE, VERFAHREN ZU IHRER HERSTELLUNG UND IHRE THERAPEUTISCHE VERWENDUNG

NOVEL (ALPHA-AMINOPHOSPHINO) PEPTIDE DERIVATIVES, METHOD FOR MAKING SAME AND THERAPEUTIC APPLICATIONS THEREOF

(84) Etats contractants désignés:
**AT BE CH DE DK ES FI FR GB GR IE IT LI LU NL PT SE**

(30) Priorité: **25.10.1996 FR 9613082**

(43) Date de publication de la demande:
**21.06.2000 Bulletin 2000/25**

(73) Titulaire: **INSTITUT NATIONAL DE LA SANTE ET DE LA RECHERCHE MEDICALE (INSERM)**
**75654 Paris Cédex 13 (FR)**

(72) Inventeurs:
- **FOURNIE-ZALUSKI, Marie-Claude**
**F-75011 Paris (FR)**
- **CHEN, Huixiong**
**F-92220 Bagneux (FR)**
- **ROQUES, Bernard, Pierre**
**F-75014 Paris (FR)**

(74) Mandataire:
**Grosset-Fournier, Chantal Catherine et al**
**Grosset-Fournier & Demachy s.a.r.l.**
**20, rue de Maubeuge**
**75009 Paris (FR)**

(56) Documents cités:
**EP-A- 0 725 075** **WO-A-91/02718**
**WO-A-95/35302**

- **CHACKALAMANNIL S ET AL: "HIGHLY POTENT AND SELECTIVE INHIBITORS OF ENDOTHELIN CONVERTING ENZYME" BIOORGANIC & MEDICINAL CHEMISTRY LETTERS, vol. 6, no. 11, 1996, pages 1257-1260, XP000676968 cité dans la demande**
- **JIRACEK J ET AL: "DEVELOPMENT OF THE FIRST POTENT AND SELECTIVE INHIBITOR OF THE ZINC ENDOPEPTIDASE NEUROLYSIN USING A SYSTEMATIC APPROACH BASED ON COMBINATORIAL CHEMISTRY OF PHOSPHINIC PEPTIDES" JOURNAL OF BIOLOGICAL CHEMISTRY, vol. 271, no. 32, 9 août 1996, pages 19606-19611, XP000677097**
- **GROBELNY D ET AL: "BINDING ENERGETICS OF PHOSPHORUS-CONTAINING INHIBITORS OF THERMOLYSIN" BIOCHEMISTRY, vol. 28, no. 12, juin 1989, pages 4948-4951, XP000009206**
- **MORGAN B P ET AL: "DIFFERENTIAL BINDING ENERGY: A DETAILED EVALUATION OF THE INFLUENCE OF HYDROGEN-BONDING AND HYDROPHOBIC GROUPS ON THE INHIBITION OF THERMOLYSIN BY PHOPHORUS-CONTAINING INHIBITORS" JOURNAL OF THE AMERICAN CHEMICAL SOCIETY, vol. 113, 2 janvier 1991, pages 297-307, XP000604955**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 009 750 B1

- **MERZ K M ET AL: "FREE ENERGY PERTURBATION SIMULATIONS OF THE INHIBITION OF THERMOLYSIN: PREDICTION OF THE FREE ENERGY OF BINDING OF A NEW INHIBITOR" JOURNAL OF THE AMERICAN CHEMICAL SOCIETY, vol. 111, no. 15, 1989, pages 5649-5658, XP002033691**

Remarques:

Le dossier contient des informations techniques présentées postérieurement au dépôt de la demande et ne figurant pas dans le présent fascicule.

## Description

La perception, la transmission et la régulation des influx nociceptifs sont sous la dépendance de plusieurs neurotransmetteurs endogènes. En 1975, Hugues et coll., *Nature,* **258,** 577, 1975 ont mis en évidence les enképhalines, deux pentapeptides isolés primitivement de cerveaux de mammifères qui sont impliqués dans la transmission des influx douloureux. Les enképhalines se lient à, au moins, deux classes de récepteurs : les sites opioïdes μ et δ (Pert, Sciences, **179**, 1011, 1973) dont les rôles et les localisations sont différents. Leurs propriétés antinociceptives ont été démontrées par Belluzi et coll., *Nature,* **260,** 625, 1976. Cependant, l'analgésie induite par administration d'enképhalines exogènes est très fugace, à cause de la métabolisation rapide de ces peptides. Des analogues d'enképhalines rendus résistants à la dégradation enzymatique par des modifications chimiques, ont été synthétisés, mais leurs effets secondaires sont analogues à ceux de la morphine.

Les enképhalines sont physiologiquement dégradées par deux types d'activités enzymatiques qui métabolisent *in vivo* les enképhalines : l'endopeptidase neutre (EC 3.4.24.11, également désignée par EPN) qui coupe la liaison $Gly^3$-$Phe^4$ et l'aminopeptidase N (EC 3.4.11.2, également désigné par APN) qui coupe la liaison $Tyr^1$-$Gly^2$ (revue dans Roques et al., *Pharmacol. Rev.,* **45,** 87-146, 1993).

On connait des prodrogues décrites dans le brevet européen EP 0 487 620 et dans Fournié-Zaluski et al., *J. Med. Chem.,* **35,** 2473, 1992, qui possèdent des activités analgésiques et antidépressives après administration intraveineuse ou par voie orale (Noble et al., *J. Pharm. Exp. Ther.*, **261,** 181, 1992 ; Baamonde et al., *Eur. J. Pharmacol.,* **216,** 157, 1992). Cependant, ces composés ne répondent pas au concept d'inhibiteurs mixtes, du fait de leur structure dans laquelle un inhibiteur d'APN et un inhibiteur d'EPN sont associés par un pont disulfure. Ces composés sont ensuite réduits par les réductases cérébrales et agissent chacun sur leur cible particulière.

D'après la demande de brevet WO 95/35302 et *Bioorganic & Medicinal Chemistry Letters,* Vol.6, No. 11, pp 1257-1260, 1996 on connaît certains dérivés de l'acide phosphinique possédant respectivement une activité inhibitrice de l'enzyme de conversion de l'endotheline (ECE) et une activité inhibitrice mixte de l'enzyme de conversion de l'angiotensine (ACE) et de l'endopeptidase neutre (EPN). Ces composés sont utiles dans le traitement de maladies cardiovasculaires.

L'un des objets de l'invention est de fournir de nouveaux composés, qui se comportent comme de véritables inhibiteurs mixtes d'APN et d'EPN, capables d'inhiber conjointement les deux activités enzymatiques responsables de la dégradation des enképhalines et de manifester leurs propriétés pharmacologiques après injection intraveineuse ou sous cutanée, ou par voie orale (per os).

Ces composés présentent certaines propriétés des substances morphiniques, en particulier l'analgésie, les effets bénéfiques sur le comportement (antidépresseurs, sédatifs, anxiolytiques, désinhibiteurs et promnésiques), et les effets périphériques (antidiarréiques, antitussifs, hypotenseurs, anti-inflammatoires...). De plus, un avantage de ces composés est qu'ils ne présentent aucun des effets néfastes des morphiniques (tolérance, dépendance physique et psychique, dépression respiratoire, stase intestinale...).

La présente invention a pour objet des composés dérivés d'(α-aminophosphino)péptides de formule générale (I)

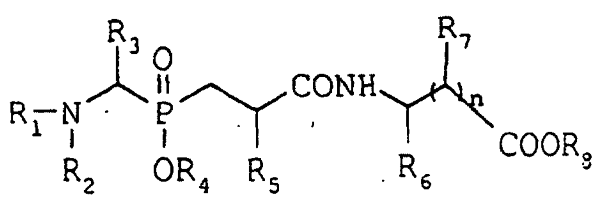

(I)

dans laquelle

- $R_1$ et $R_2$ représentent chacun un atome d'hydrogène ou bien $R_1$ et $R_2$, pris ensemble, forment un groupe insaturé de formule R'(R")C=, dans laquelle,
  - R' représente un groupe phényle monosubstitué en position 2 par un groupe hydroxy ou bien un groupe phényle disubstitué, en position 2, par un groupe hydroxy et, en position 4 ou 5, soit par un atome d'halogène soit par un groupe nitro, soit par un groupe hydroxy, soit par un groupe alcoxy $-OR_9$,
  - R" représente un groupe phényle, un groupe phényle substitué par 1 à 5 atomes d'halogène ou un groupe hétérocyclique aromatique,

par la suite, les termes $R_9$ et $R_{10}$, utilisés pour la définition des radicaux, représentent chacun un groupe alkyle de 1 à 6 atomes de carbone,

$R_3$ représente

- un atome d'hydrogène,
- un groupe alkyle ou un groupe alkényle de 1 à 6 atomes de carbone, ces deux derniers groupes pouvant être substitués par :
    - un groupe hydroxy ou un groupe alcoxy $-OR_9$,
    - un groupe phényle ou un groupe benzyle,
    - un groupe sulfanyle, un groupe alkylsulfanyle $-SR_9$ ou un groupe alkylsulfanyle oxydé sur l'atome de soufre $-S(O)R_9$,
    - un groupe amino, un groupe $-NHR_9$ ou $-NR_9R_{10}$, éventuellement oxydé sur l'atome d'azote ou,
    - un groupe guanidino $H_2N-C(=NH)-NH-$,
- un groupe cycloalkyle ou cycloalkylméthyle,
- un groupe phényle, un groupe benzyle, pouvant être substitués sur le groupe phényle par 1 ou 2 des substituants suivants :
    - un atome d'halogène,
    - une groupe hydroxy, un groupe alcoxy $-OR_9$,
    - un groupe alkylsulfanyle $-SR_9$ ou un groupe alkylsulfanyle oxydé sur l'atome de soufre,
    - un groupe amino ou un groupe $-NHR_9$ ou $-NR_9R_10$ éventuellement oxydé sur l'atome d'azote,
    - un groupe nitro,
    - un groupe phényle,
    - un groupe alkyle de 1 à 4 atomes de carbone,
- un groupe méthyle substitué par un groupe hétérocyclique aromatique ou saturé, les hétéroatomes pouvant être oxydés sous forme de N-oxyde ou de S-oxyde,

$R_4$ représente

- un atome d'hydrogène,
- un groupe alkyle ou alkényle de 1 à 6 atomes de carbone,
- un groupe cycloalkyle, un groupe cycloalkylalkyle,
- un groupe phényle, un groupe benzyle, pouvant être substitués sur le groupe phényle par 1 ou 2 des substituants suivants :
    - un groupe alkyle de 1 à 6 atomes de carbone,
    - un atome d'halogène,
    - un groupe hydroxy ou un groupe alcoxy $-OR_9$,
    - un groupe trifluorométhyle,
    - un groupe nitro,

$R_5$ représente

- un atome d'hydrogène,
- un groupe alkyle ou un groupe alkényle de 1 à 6 atomes de carbone, ces deux derniers groupes pouvant être substitués par :
    - un groupe hydroxy ou un groupe alcoxy $-OR_9$,
    - un groupe phényle ou un groupe benzyle,
    - un groupe sulfanyle, un groupe alkylsulfanyle $-SR_9$ ou un groupe alkylsulfanyle oxydé sur l'atome de soufre $-S(O)R_9$,
    - un groupe amino, un groupe $-NHR_9$ ou $-NR_9R_{10}$, éventuellement oxydé sur l'atome d'azote ou,
    - un groupe guanidino $H_2N-C(=NH)-NH-$,

- un groupe cycloalkyle ou cycloalkylméthyle,
- un groupe phényle, un groupe benzyle, pouvant être substitués sur le groupe phényle par 1 ou 2 des substituants suivants :
  - un atome d'halogène,
  - une groupe hydroxy, un groupe-alcoxy $-OR_9$,
  - un groupe alkylsulfanyle $-SR_9$ ou un groupe alkylsulfanyle oxydé sur l'atome de soufre,
  - un groupe amino ou un groupe $-NHR_9$ ou $-NR_9R_10$ éventuellement oxydé sur l'atome d'azote,
  - un groupe nitro,
  - un groupe phényle,
  - un groupe alkyle de 1 à 4 atomes de carbone,
- un groupe méthyle substitué par un groupe hétérocyclique, les hétéroatomes pouvant être oxydés sous forme de N-oxyde ou de S-oxyde,
- $R_6$ et $R_7$ représentent indépendamment l'un de l'autre
  - un atome d'hydrogène,
  - un groupe alkyle ou alkényle de 1 à 6 atomes de carbone, pouvant être substitué par :
    - > un groupe hydroxy ou un groupe alcoxy $-OR_9$,
    - > un groupe sulfanyle, un groupe alkylsulfanyle $-SR_9$ ou un groupe alkylsulfanyle oxydé sur l'atome de soufre $-S(O)R_9$,
    - > un groupe amino ou un groupe alkylamino $-NHR_9$,
    - > un groupe guanidino $H_2N-C(=NH)-NH-$ ou,
    - > un groupe carboxy ou un groupe alkyloxycarbonyl $-COOR_9$,
  - un groupe phényle, un groupe benzyle, pouvant être substitués sur le groupe phényle par 1 ou 2 substituants suivants :
    - > un atome d'halogène,
    - > un groupe phényle,
    - > un groupe hydroxy ou un groupe alcoxy $-OR_9$,
    - > un groupe alkylsulfanyle $-SR_9$ ou un groupe alkylsulfanyle oxydé sur l'atome de soufre $-S(O)R_9$,
- $R_6$ et $R_7$ représentent ensemble un cycle saturé ou insaturé à 5 ou 6 chaînons, comprenant 1 ou 2 hétéroatomes pris parmi l'oxygène, le soufre et l'azote,
- $R_8$ représente,
  - un atome d'hydrogène,
  - un groupe alkyle ou alkényle de 1 à 6 atomes de carbone,
  - un groupe phényle, un groupe benzyle,
- n est égal à 0 ou 1,

à l'exception de l'hydrochlorure de *N*- [2-[[(aminométhyl) (méthoxy)phosphinyl]méthyl]-4-méthyl-1-oxopentyl]-(1,1'-biphényl-4-yl)-*L*-alaninate de méthyle.

Dans le cadre de l'invention les termes ci-après ont les significations suivantes :

- un groupe alkyle est une chaîne hydrocarbonée, saturée, linéaire ou ramifiée,
- un groupe alkényle est une chaîne hydrocarbonée linéaire ou ramifiée, comportant une insaturation,
- un groupe cycloalkyle est une chaîne hydrocarbonée cyclique comprenant 3 à 7 atomes de carbone,
- un groupe cycloalkylalkyle est un groupe cycloalkyle lié à un groupe alkyle, ce groupe alkyle comprenant 1 à 6 atomes de carbone,
- un groupe cycloalkylméthyl est un groupe cycloalkyle lié à un groupe méthyle,
- un groupe hétérocyclique est une chaîne hydrocarbonée cyclique, aromatique ou non, à 5 ou 6 chaînons, comprenant 1 ou 2 hétéroatomes pris parmi les atomes d'oxygène, de soufre ou d'azote,

Dans lé cadre de l'invention, les atomes d'halogène sont préférentiellement le chlore et le fluor.

Lorsqu'un groupe phényle est substitué par un groupe phényle, il l'est alors préférentiellement en position 4 pour former un groupe biphényle (s'écrit également : [1,1'-biphényl]).

**[0011]** - La présente invention a également pour objet les sels d'addition aux acides pharmacologiquement acceptables des composés de formule (I) pour lesquels $R_1$ et $R_2$ sont des atomes d'hydrogène.

Une catégorie de composés préférés selon l'invention sont ceux pour lesquels les radicaux de la formule (I) ont les significations suivantes :

- $R_1$, $R_2$, $R_4$, $R_8$ représentent des atomes d'hydrogène,
- n est égal à 0,
- $R_3$ représente
  - un groupe alkyle de 1 à 6 atomes de carbone, pouvant être substitué par un groupe alcoxy $-OR_9$, un groupe sulfanyle ou un groupe alkylsulfanyle $-SR_9$,
  - un groupe phényle, un groupe benzyle, pouvant être substitués sur le groupe phényle par un atome d'halogène, un groupe alkyle de 1 à 4 atomes de carbone, un groupe alcoxy $-OR_9$ ou un groupe alkylsulfanyle $-SR_9$,
- $R_5$ représente
  - un groupe alkyle de 1 à 6 atomes de carbone, pouvant être substitué par un groupe alcoxy $-OR_9$, un groupe sulfanyle ou un groupe alkylsulfanyle $-SR_9$,
  - un groupe phényle, un groupe benzyle, pouvant être substitués sur le groupe phényle par un atome d'halogène, un groupe alkyle de 1 à 4 atomes de carbone, un groupe alcoxy $-OR_9$ ou un groupe alkylsulfanyle $-SR_9$,
  - un groupe biphénylméthyle,
- $R_6$ représente
  - un groupe alkyle de 1 à 6 atomes de carbone, pouvant être substitué par un groupe alcoxy $-OR_9$, un groupe sulfanyle ou un groupe alkylsulfanyle $-SR_9$,
  - un groupe phényle, un groupe benzyle, pouvant être substitués sur le groupe phényle par un atome d'halogène, un groupe alkyle de 1 à 4 atomes de carbone, un groupe alcoxy $-OR_9$ ou un groupe alkylsulfanyle $-SR_9$,
  - un groupe biphénylméthyle,

Les composés de formule (I) peuvent possèder de 1 à 5 atomes chiraux. Les composés de l'invention peuvent exister sous différentes formes isomères y compris sous forme d'énantiomères et de diastéréoisomères. La présente invention comprend ces différentes formes ainsi que leurs mélanges, y compris les mélanges racémiques.

Le carbone portant le radical $R_6$, lorsque $R_6$ est différent de l'atome d'hydrogène, est avantageusement de configuration absolue (*S*).

Les composés de l'invention de formule (I) peuvent être préparés selon les procédés décrits dans les annexes 1, 2 et 3.

Dans la description du procédé, les radicaux $A_1$ et $A_2$ et $A_3$ ont les significations suivantes :

- $A_1$ représente un groupe biphénylméthyle, un groupe tert-butoxycarbonyle, un groupe benzyloxycarbonyle ou un groupe fluorénylméthoxycarbonyle,
- $A_2$ représente un atome d'hydrogène, un groupe alkyle tel que méthyle ou éthyle, ou un groupe benzyle,
- $A_3$ représente un groupe méthyle, éthyle, *tert*-butyle ou benzyle.

Les composés de formule (Ia), (Ib), (Ic), qui sont des composés de formule (I) selon l'invention, sont préparés selon le procédé représenté en annexe 1. $R_3$, $R_4$, $R_5$, $R_6$, $R_7$, R' et R'' ont les significations données dans la formule (I). Selon ce procédé, on fait réagir un dérivé de l'acide hydroxyphosphinylpropanoïque de formule (IVb) avec un dérivé d'acide aminé de formule (III), en présence de benzotriazol-1-yloxy-tris(dimethylamino)phosphonium hexafluorophosphate (BOP), dans un solvant organique tel que le diméthylformamide. Le composé de formule (III) peut être utilisé

sous forme de sel, tel que le sel de l'acide *p*-toluènesulfonique. Il est alors particulièrement avantageux d'opérer en présence d'une base telle qu'une amine tertiaire comme la diisopropyléthylamine. Cette réaction permet l'obtention d'un composé de formule (IIa).

Dans le cas où $R_5$ représente dans le produit final de formule (I) un groupe biphénylméthyle, on procède selon une variante lors de cette étape qui consiste à effectuer le couplage décrit ci-dessus dans les mêmes conditions opératoires, à partir du composé de formule (IVb) et du composé de formule (III), dans laquelle le radical $R_5$ représente tout d'abord un groupe (4-bromophényl)méthyle. On fait réagir le composé de formule (IIa) alors obtenu avec de l'acide phénylboronique dans un solvant, tel qu'un mélange toluène/méthanol et en présence de tétrakis(triphénylphosphine) palladium et de carbonate de sodium pour obtenir le composé de formule (IIa), dans laquelle $R_5$ est un groupe biphénylméthyl.

Le composé de formule (IIa) est utilisé pour préparer le composé de formule (Ia), dans lequelle les trois fonctions amine, phosphinate et carboxylate sont déprotégées simultanément ou successivement. La fonction carboxy peut être déprotégée pour donner un composé de formule (IIb), par exemple par saponification. La fonction phosphinate peut être déprotégée pour donner un composé de formule (IIc). La fonction amine peut être déprotégée pour donner un composé de formule (la), par exemple par hydrogénation catalytique ou hydrolyse acide. Il s'agit de l'alternative C, représentée en annexe 1.

Selon un mode de préparation alternatif, on peut obtenir directement le composé de formule (Ia) à partir du composé de formule (IIa) par hydrogénation catalytique, en particulier lorsque $A_1$ représente un groupe benzyloxycarbonyle, $A_2$ représente un groupe benzyle et $A_3$ représente un groupe benzyle.
Il s'agit de l'alternative B, représentée en annexe 1.

Pour préparer le composé de formule (Ib), dans lequel $R_4$ et $R_8$ sont différent de l'atome d'hydrogène, on procède à l'estérification du composé de formule (IIc), dans laquelle la fonction amine est protégée, selon des méthodes connues de l'homme de l'art, ce qui revient à introduire les radicaux $R_4$ et $R_8$, respectivement sur la fonction phosphinate et sur la fonction carboxylate. On peut ainsi condenser sur un composé de formule (IIc) un alcool $R_4OH$, dans lequel $R_4$ a l'une des significations données dans la formule(I) à l'exception de l'hydrogène, en présence d'un agent de couplage tel que le dicyclohydroxylcarbodiimide (DCC) et d'une base telle que la diméthylaminopyridine (DMAP), sur ce composé de formule (IIc). Enfin on déprotège la fonction aminé.

Selon un mode de préparation alternatif du composé de formule (Ib), dans laquelle les radicaux $R_4$ et $R_8$ représentent chacun l'un des substituants possibles respectivement de $A_2$, à l'exclusion de l'atome d'hydrogène, et de $A_3$, on peut obtenir directement le composé de formule (Ib), en déprotégeant uniquement la fonction aminé du composé de formule (IIa). Il s'agit de l'alternative A, représentée en annexe 1.

Pour préparer le composé de formule (Ic), on condense une cétone R'(R")C=O sur un composé de formule (Ib) si $R_4$ et $R_8$ sont différents de l'atome d'hydrogène et sur un composé de formule (Ia) si $R_4$ et $R_8$ représentent un atome d'hydrogène, ces cétones R'(R")C=O étant obtenues par réarrangement de Fries des esters correspondants $R''CO_2R'$.

Les composés de formule (III), dans laquelle n peut être égal à 0 ou 1, $R_6$ et $R_7$ prenant l'une des significations données dans la formule (I), représentent un $\alpha$ ou un $\beta$ aminoacide naturel ou non. Ils peuvent être synthétisés selon les méthodes classiques bien connues de l'homme de l'art.

Les composés de formule (IVb) peuvent être préparés selon les procédés décrits dans les annexes 2 et 3. L'obtention du composé de formule (IVb) à partir du composé de formule (IVa) est une saponification classique.

La dernière étape du procédé est représentée dans le schéma 1 de l'annexe 2. Selon cette dernière étape du procédé, on additionne un dérivé de l'acide phosphonique de formule (VIb) sur un dérivé de l'ester acrylique de formule (V), en présence de *N*,*O*-bis(triméthylsilyl)acétamide, sans solvant ou dans un solvant organique inerte tel que l'acétonitrile.

Le composé de formule (VIb) est obtenu par synthèse indirecte ou directe, représentée dans le schéma 2 de l'annexe 2 :

- par la voie indirecte, le procédé consiste à traiter de la diphénylméthylamine par de l'acide phosphoneux et à faire réagir la diphénylméthylamine sous-forme de sel de l'acide phosphoneux obtenu, avec un aldéhyde $R_3CHO$ dans de l'éthanol anhydre pour obtenir un composé de formule (X). La fonction amine de ce composé de formule (X) est ensuite déprotégée dans l'eau éventuellement acidifiée par un acide minéral, tel que l'acide chlorhydrique ou l'acide bromhydrique, puis le composé obtenu est traité par de l'oxyde de propylène pour conduire aux composés de formule (VII), que l'on acyle enfin pour obtenir un composé de formule (VIa),
- par la voie directe, le procédé consiste à traiter le chlorhydrate de diphénylméthylamine par l'acide phosphoneux et un aldéhyde $R_3CHO$, cette réaction étant effectuée dans un mélange d'éthanol et d'eau au reflux.

Les composés de formule (V) peuvent être obtenus par deux méthodes, qui sont représentées dans l'annexe 3, respectivement aux schémas 3 et 4 :

- selon le premier procédé (schéma 3), on fait réagir un halogénure, et de préférence un bromure, d'alkyle ou d'aralkyle $R_5X$ avec le triéthylphosphonoacétate, en présence d'hydrure de sodium pour obtenir un composé de formule (VIII) que l'on fait réagir sur du formaldéhyde, en présence de carbonate de potassium pour obtenir un composé de formule (V).

- selon le second procédé (schéma 4), on obtient les composés de formule (V) par réaction de Mannich sur un monoester d'un acide malonique de formule (IX).

Un autre aspect de l'invention est relatif aux composés de formule (IIa), utiles notammant en tant qu'intermédiaires de synthèse pour la préparation des composés de formule (I). Ils peuvent également se présenter sous forme d'isomères, y compris sous forme d'énantiomères et de diastéréoisomères et de mélanges de ces différentes formes ainsi que sous forme de sels d'addition.
Toutefois, les composés suivants, décrits dans Morgan B P et al., "differential binding energy: a detailed evalutation of the influence of hydrogen-bonding and hydrophobic groups on the inhibition of thermolysin by phosphorus-containing inhibitors, *Journal of the American Chemical Society* (1991), 113, 297-307, sont exclus :

le *N*-[2-[[méthoxy[[[(phénylméthoxy)carbonyl]amino]méthyl] phosphinyl]méthyl]-4-méthyl-1-oxopentanyl]-*L*-alaninate de méthyle,
le *N*-[-[[méthoxy[[[(phénylméthoxy)carbonyl]amino]méthyl] phosphinyl]méthyl]-4-méthyl-1-oxopentanyl]-*L*-glycinate de méthyle,
le *N*-[-[[méthoxy[[[(phénylméthoxy)carbonyl]amino]méthyl] phosphinyl]méthyl]-4-méthyl-1-oxopentanyl]-*L*-phénylalaniante de méthyle et,
le *N*-[-[[méthoxy[[[(phénylméthoxy)carbonyl]amino]méthyl] phosphinyl]méthyl]-4-méthyl-1-oxopentanyl]-*L*-leucinate de méthyle.

Les composés de formule générale (I), ainsi obtenus, sont sous forme d'isomères, y compris sous forme d'énantiomères, de diastéréoisomères et de mélanges de ces différentes formes, y compris de mélanges racémiques.
Les composés de formule (I), optiquement purs, sont obtenus par séparation par HPLC semi-préparative (Chromasil $C_8$, 10 mm, 20 x 250 mm, acétonitrile-eau, 15 ml/min).
Ils peuvent également être obtenus par dédoublement, à partir d'une amine chirale, du dérivé de l'acide phosphinique de formule (VIb), dans laquelle $A_2$=H puis par addition de Michaël diastéréosélective, en présence d'inducteurs chiraux, qui conduit aux composés de formule (IV), qui ont une stéréochimie parfaitement définie.
Les exemples suivants ont pour but d'illustrer la préparation de quelques composés de l'invention. Les analyses élémentaires et les spectres RMN confirment les structures des composés obtenus.
Dans les noms des composés, 1e tiret "-" fait partie du mot, et le tiret"_" ne sert que pour la coupure en fin de ligne ; il est à supprimer en l'absence de coupure et ne doit être remplacé ni par un tiret normal ni par un espace.
Dans les exemples suivants, le mélange racémique obtenu pour chacun des composés peut être séparé sur colonne préparative Chromasil $C_8$, 10mm, 20 x 250 mm, 15 ml/min) avec un mélange acétonitrile / eau. Les acides aminés utilisés lors de ces synthèses ont une configuration absolue (*S*), on obtient donc un mélange de quatre diastéréoisomères. Par convention on appelle ces diastéréoisomères A, B, C, D par ordre de temps de rétention croissant. Les temps de. retention des composés sont rassemblés dans le tableau.

<u>Exemple 1</u> : <u>Chlorhydrate de *N*-[2-[[(1-amino-2-phényléthyl) hydroxyphosphinyl]méthyl]-1-oxo-3-phénylpropyl]-*L*-phénylalanine</u>

1.1. Phosphinate de α-phénylbenzèneméthanamine

A une solution de 8 g (121,2 mmol) de l'acide phosphoneux dans 30 ml d'éthanol anhydre, refroidi à 0°C, sont ajoutés goutte à goutte 22,21 g (121,2 mmol) de diphénylméthylamine en veillant à ce que la température ne dépasse pas 25°C. Il se forme, après addition, un précipité blanc auquel on ajoute 200 ml de diéthyléther. Le précipité est filtré, rincé au diéthyléther puis séché.
On récupère 22,66 g de produit (rendement = 98,2 %).
Point de fusion : 176-177°C.

1.2. Acide [1-[(diphénylméthyl)amino]-2-phényléthyl] phosphinique

Une solution de 21 g (84,3 mmol) de phosphinate de a-phénylbenzèneméthanamine dans 60 ml d'éthanol anhydre est porté au reflux. 20,25 g (168,5 mmol) de phénylacétaldéhyde dans 20 ml d'éthanol anhydre sont ajoutés

goutte à goutte. Il se forme un précipité blanc abondant. Après addition, on maintient le reflux pendant 2,5 heures. Après refroidissement, on ajoute 100 ml d'acétone. Le précipité obtenu est filtré et lavé avec le même solvant.
On récupère 18,6 g de produit (rendement = 62,9 %).
Point de fusion : 211°C.

1.3. Acide (1-amino-2-phényléthyl)phosphinique

Un mélange de 14,6 g (41,6 mmol) d'acide [1-[(diphénylméthyl) amino]-2-phényléthyl]phosphinique et de 94 ml de l'acide bromhydrique aqueux à 48 % est porté à reflux pendant 2 heures. Deux phases apparaissent. Le mélange est mis à sec par évaporation sous vide à chaud puis repris dans 94 ml d'eau. On lave trois fois la phase aqueuse au diéthyléther. La phase aqueuse, à nouveau évaporée, conduit au bromhydrate de l'acide (1-amino-2-phényléthyl)phosphinique, qui est repris dans 58 ml d'éthanol absolu. On ajoute 20,4 ml d'oxyde de propylène, goutte à goutte, sous agitation, à 0°C. On observe la formation d'un précipité blanc abondant qui est filtré et lavé au diéthyléther.
On récupère 6,77 g de produit (rendement = 87,9 %).
Point de fusion : 226-227°C.

1.4. Acide [2-phényl-1-[[(phénylméthoxy)carbonyl]amino]éthyl] phosphinique

A une solution de 7 g (37,8 mmol) d'acide (1-amino-2-phényléthyl)phosphinique dans 86 ml d'un mélange de dioxane et d'eau, en présence d'un équivalent de soude (1,51 g), on ajoute, à 0°C, goutte à goutte, et simultanément une solution de 7,55 g (44,26 mmol) de chloroformiate de benzyle dans 21 ml de dioxane et, de manière à maintenir le pH de la réaction autour de 9, une solution de 2,42 g de soude dans 21 ml d'eau. A la fin de l'addition, le mélange est maintenu à une température voisine de 20°C pendant 2 heures. On lave ensuite la phase aqueuse par 3 fois 20 ml de diéthyléther. On acidifie la phase aqueuse, à 0°C, sous vive agitation, avec 27 ml d'une solution d'acide chlorhydrique 6N. Le précipité blanc abondant formé est filtré, lavé à l'eau et séché.
On récupère 12,08 g de produit (rendement = 83,7%).
Point de fusion : 135-136°C.

1.5. α-méthylènebenzènepropanoate de méthyle

A une solution de 20 g (123,5 mmol) d'acide α-méthylènebenzènepropénoïque dans 12 ml de méthanol, refroidi à 0°C, on ajoute, goutte à goutte, 2,7 ml de chlorure de thionyle. Le mélange est porté à température de reflux pendant 6 heures, puis le solvant est évaporé sous pression réduite. Le résidu est repris dans 30 ml d'acétate d'éthyle. Cette solution est lavée avec de l'hydrogénocarbonate de sodium 10% puis par de l'eau. La phase organique est séchée sur sulfate de sodium, filtrée et évaporée sous pression réduite. On purifie le résidu par chromatographie sur gel de silice en éluant par un mélange acétate d'éthyle / hexane : 1 / 3.
On récupère 21,3 g de produit sous forme d'huile (rendement = 98 %).

1.6. 3-[Hydroxy[2-phényl-1-[[(phénylméthoxy)carbonyl]amino] éthyl]phosphinyl]-2-(phénylméthyl)propanoate de méthyle

Une solution de 1 g (3,31 mmol) de l'acide [2-phényl-1-[[(phénylméthoxy) carbonyl] amino] éthyl]phosphinique, 0,66 g (3,75 mmol) de α-méthylènebenzènepropanoate de méthyle et 3,1 ml de *N,O*-bis(triméthylsilyl)acétamide est portée à 60°C pendant 24 heures. Après refroidissement, on ajoute 60 ml d'un mélange acétate d'éthyle / eau : 1 / 1. La phase aqueuse est extraite par de l'acétate d'éthyle. L'ensemble des phases organiques est lavé à l'eau, séché sur sulfate de sodium, filtré et évaporé sous vide. On purifie par recristallisation dans un mélange d'acétate d'éthyle et d'hexane.
On récupère 1,04 g de produit (rendement = 67%).
Point de fusion : 161-163°C.

1.7. Acide 3-[hydroxy[2-phényl-1-[[(phénylméthoxy)carbonyl] amino]éthyl]phosphinyl]-2-(phénylméthyl)propanoïque

A une solution de 1 g (2,02 mmol) de 3-[Hydroxy[2-phényl-1-[[(phénylméthoxy)carbonyl]amino]éthyl]phosphinyl]-2-(phénylméthyl)propanoate de méthyle dans 20 ml de méthanol, on ajoute 20 ml de soude 1N. Après 10 heures d'agitation, on acidifie le milieu avec de l'acide chlorhydrique 2N, on évapore ensuite le méthanol et extrait trois fois avec de l'acétate d'éthyle. L'ensemble des phases organiques est lavé avec de l'eau, séché sur sulfate de sodium et évaporé.
On récupère 0,97 g de produit (rendement = 100%).

Point de fusion : 178-179°C.

1.8. *N*-[2-[[Hydroxy[2-phényl-1-[[(phénylméthoxy)carbonyl] amino]éthyl]phosphinyl]méthyl]-1-oxo-3-phénylpropyl]-*L*-phénylalaninate de phénylméthyle

A 200 mg (0,42 mmol) d'acide 3-[hydroxy[2-phényl-1-[[(phénylméthoxy)carbonyl]amino]éthyl]phosphinyl]-2-(phénylméthyl)propanoïque, 178 mg (0,42 mmol) de *L*-phénylalaninate de phénylméthyle sous forme de sel de l'acide *p*-toluènesulfonique et 384 mg (0,87 mmol) de BOP dans 0,8 ml de diméthylformamide, on ajoute, sous agitation à température voisine de 20 °C, 0,7 ml de diisopropyléthylamine. Après 30 minutes de réaction à cette température, on évapore le diméthylformamide sous pression réduite, on ajoute 12 ml d'acide chlorhydrique 1N au résidu huileux obtenu. Le précipité est filtré, lavé avec de l'eau et séché.
On récupère 210 mg de produit (rendement = 70,3%).
Point de fusion : 173-176°C.

1.9. Chlorhydrate de *N*- [2-[[(1-amino-2-phényléthyl)hydroxy phosphinyl]méthyl]-1-oxo-2-phénylpropyl]-*L*-phénylalanine

A une solution de 140 mg (0,19 mmol) de *N*-[2-[[Hydroxy[2-phényl-1-[[1-(phénylméthoxy)carbonyl)amino] éthyl]phosphinyl] méthyl]-1-oxo-3-phénylpropyl]-*L*-phénylalaninate de phénylméthyle dans 20 ml de méthanol, on ajoute, à 0°C, 20 mg de Palladium sur charbon. Le mélange est ensuite soumis à une hydrogénolyse à une température voisine de 20°C, sous agitation, en présence d'un courant d'hydrogène. Après 2 heures, le mélange réactionnel est filtré, rincé avec du méthanol. Au filtrat, on ajoute un équivalent d'acide chlorhydrique 6N. La solution est évaporée sous pression réduite.
On récupère 87 mg de produit (rendement = 84,1%).
Point de fusion : 220°C (décomposition).

Exemple 2 : Bromhydrate de *N*-[2-[[(1-amino-2-phényléthyl) hydroxyphosphinyl]méthyl]-1-oxo-3-phénylpropyl]-*L*-alanine

2.1. *N*-[2-[[Hydroxy[2-phényl-1-[[(phénylméthoxy)carbonyl] amino]éthyl]phosphinyl]méthyl]-1-oxo-3-phénylpropyl]-L-alaninate de phénylméthyle

On procède selon les conditions opératoires décrites en 1.8 à partir de 300 mg (0,62 mmol) d'acide 3-[hydroxy [2-phényl-1-[[(phénylméthoxy)carbonyl]amino]éthyl]phosphinyl]-2-(phénylméthyl)propanoique synthétisé en 1.7., et 219 mg (0,62 mmol) d'alaninate de phénylméthyle sous forme de sel de l'acide *p*-toluènesulfonique.
On récupère 288 mg de produit (rendement = 71,9 %).
Point de fusion : 171-174°C

2.2. *N*-[2-[[Hydroxy[2-phényl-1-[[(phénylméthoxy)carbonyl] amino]éthyl]phosphinyl]méthyl]-1-oxo-3-phénylpropyl]-L-alanine

On procède selon les conditions opératoires décrites en 1.7. à partir de 100 mg (0,16 mmol) de *N*-[2-[[hydroxy [2-phényl-1-[[(phénylméthoxy)carbonyl]amino]éthyl]phosphinyl]méthyl]-1-oxo-3-phénylpropyl]-L-alaninate de phénylméthyle.
On récupère 83,7 mg de produit (rendement = 97,4 %).
Point de fusion : 192-195°C.

2.3. Bromhydrate de *N*-[2-[[(1-amino-2-phényléthyl)hydroxy_ phosphinyl]méthyl]-1-oxo-3-phénylpropyl]-*L*-alanine

A une solution de 50 mg (0,09 mmol) de *N*-[2-[[Hydroxy[2-phényl-1-[[(phénylméthoxy)carbonyl]amino]éthyl] phosphinyl] méthyl]-1-oxo-3-phénylpropyl]-L-alanine dans 2,5 ml de dichlorométhane, refroidie à -10°C, on ajoute, sous azote, 0,45 ml de tribromure de bore en solution 1M dans le dichlorométhane (0,45 mmol). Le mélange est agité pendant une heure à -10°C puis pendant 2 heures à une température voisine de 20°C. On ajoute 3 ml d'eau et évapore-le dichlorométhane. La phase aqueuse est lavée avec du diéthyléther et évaporée à sec. Le produit est repris dans l'eau puis lyophilisé.
On récupère 45 mg de produit (rendement = 100%).
Point de fusion : 210°C (décomposition).
*Sont obtenus de manière analogue :*

Les composés n°3,4,5 à partir des produits de départ adéquats. Les carboxylates d'α-aminoacides utilisés proviennent soit des acides aminés naturels, soit sont synthétisés selon des méthodes connues de l'homme de l'art.

Exemple 3 : Bromhydrate de l'acide (*S*)-β-[[3-[(1-amino-2-phényléthyl)hydroxyphosphinyl]-1-oxo-2-(phénylméthyl)propyl) amino]benzènebutanoïque

3.1. (*S*)-[3-Diazo-2-oxo-1-phénylméthyl)propyl]carbamate de 1,1-diméthyléthyle

A une solution de 10 g (37,69 mmol) de *N*-[(1,1-diméthyl_ éthoxy)carbonyl]-*L*-phénylalanine dans 56 ml de tétrahydrofurane anhydre refroidie à une température de -20°C, on ajoute, sous argon, 3,7 ml de N-méthylmorpholine puis, goutte à goutte, 4,4 ml de chloroformiate d'isobutyle en 30 minutes puis le mélange est filtré. Au filtrat, on ajoute l'excès de méthanediazonium en solution dans le diéthyléther. Le mélange est agité, sous argon, pendant 30 minutes à -20°C puis pendant 2 heures à une température voisine de 20°C. L'excès de méthanediazonium est enlevé par passage d'argon. Le solvant est évaporé sous pression réduite. Le résidu est repris dans de l'acétate d'éthyle, lavé successivement avec de l'eau, de l'acide citrique à 10%, de l'hydrogénocarbonate de sodium 1N, de l'eau, séché sur sulfate de sodium et filtré. Le solvant est évaporé sous pression réduite.
On récupère 10,7 g de produit (rendement = 98%).
Point de fusion : 95-96°C.

3.2. *(S)*-β-[[(1, 1-diméthyléthoxy)carbonyl]amino] benzènebutanoate de méthyle

A une solution de 10,7 g (36,98 mmol) de (*S*)-[3-Diazo-2-oxo-1-(phénylméthyl)propyl]carbamate de 1,1-diméthyléthyle dans 110 ml de méthanol, on ajoute, sous azote, 12 ml d'une solution de benzoate d'argent (1,15 g dans 23 ml de triéthylamine). Le mélange est agité à une température voisine de 20 °C pendant 30 minutes. On ajoute encore 5,5 ml de benzoate d'argent dans le même solvant. Le mélange est agité ensuite pendant 2 heures. On ajoute 35 ml de chlorure de sodium saturé, célite et charbon activé. Après filtration, le solvant est évaporé sous pression réduite. Le résidu est repris dans de l'acétate d'éthyle, lavé successivement avec de l'eau, de l'hydrogénocarbonate de sodium 1N, de l'acide chlorhydrique 1N, de l'eau, séché sur sulfate de sodium et filtré. Le solvant est évaporé sous pression réduite. On purifie par recristallisation dans l'hexane.
On récupère 8,4 g de produit (rendement = 78%).
Point de fusion : 51-52°C.

3.3. (*S*)-β-Aminobenzènebutanoate de méthyle

A une solution de 3 g (10,24 mmol) de *(S)*-β-[[(1,1-diméthyl_ éthoxy)carbonyl]amino]benzènebutanoate de méthyle dans 1,2 ml de dichlorométhane, on ajoute 1,2 ml de l'acide trifluoroacétique. Le mélange est agité à une température voisine de 20°C pendant une heure. Le solvant est évaporé à sec sous pression réduite. Le résidu est repris dans 40 ml de dichlorométhane. On ajoute de la soude 1N jusqu'à pH=8. La phase organique est lavée avec de l'eau, séchée sur sulfate se sodium et filtrée. Le solvant est évaporé sous pression réduite.
On récupère 1,89 g de produit (rendement = 95,6%).
$R_f$ (acétate d'éthyle) : 0,26

3.4. Bromhydrate de l'acide (*S*)-β-[3-[[(1-amino-2-phényléthyl)hydroxyphosphinyl]-1-oxo-2-(phénylméthyl)propyl] amino]benzènebutanoïque

On procède selon les conditions opératoires décrites en 1.8. partir de l'acide 3-[hydroxy[2-phényl-1-[[(phénylméthoxy) carbonyl]amino]éthyl]phosphinyl]-2-(phénylméthyl)propanoïque et du (*S*)-β-Aminobenzènebutanoate de méthyle (rendement = 71,3%), puis, on procède successivement, en utilisant à chaque fois comme produit de départ le composé synthétisé à l'étape précédente, selon les conditions opératoires décrites en 1.7. (rendement = 94%), puis en 2.3. (rendement = 100%). Point de fusion : 240°C (décomposition).

Exemple 4 : Bromhydrate de *N*-[2-[[(1-amino-2-phényléthyl) hydroxyphosphinyl]méthyl]-4-méthyl-1-oxopentyl]-*L*-phénylalanine

4.1. 2-(diéthoxyphosphinyl)-4-méthylpentanoate d'éthyle

A une solution de 31 ml (156 mmol) de (diéthylphosphinyl) acétate d'éthyle dans 49 ml de diméthylformamide, on ajoute, à -10°C, 5,16 g (172 mmol) d'hydrure de sodium à 80%. Après 15 minutes, on ajoute, goutte à goutte 17,6

ml (162 mmol) de bromure d'isobutyle. Le mélange réactionnel est porté à une température voisine de 20°C et agité à cette température pendant la nuit. Le solvant est évaporé sous pression réduite. Le résidu est repris dans de l'acétate d'éthyle, lavé avec de l'eau, séché sur sulfate de sodium et filtré. Le solvant est évaporé sous pression réduite. On purifie par chromatographie sur gel de silice en éluant par un mélange acétate d'éthyle / heptane : 1 / 1.
On récupère 23,7 g de produit sous forme d'huile (rendement = 54,3%).
Rf (heptane / acétate d'éthyle : 3,5 / 6,5) : 0,39.

4.2. 4-Méthyl-2-méthylènepentanoate d'éthyle

Un mélange de 19 g (67,9 mmol) de 2-(diéthoxyphosphinyl)-4-méthylpentanoate d'éthyle, de 27 ml (360,3 mmol) de formaldéhyde et de 28 g (202,6 mmol) de carbonate de potassium est porté à reflux pendant 3 heures. On ajoute un mélange d'eau et de diéthyléther. La phase aqueuse est extraite avec du diéthyléther. L'ensemble des phases organiques est lavé avec de l'eau, séché sur sulfate de sodium, filtré et évaporé sous pression réduite. Le résidu est distillé sous pression réduite.
On récupère 6,7 g de produit sous forme d'huile
(rendement = 63,3%).

4.3. 2-[[Hydroxy[2-phényl-1-[[(phénylméthoxy)carbonyl]amino] éthyl]phosphinyl]méthyl]-4-méthylpentanoate d' éthyle

A une solution de 1 g (3,13 mmol) d'acide [2-phényl-1-[[(phénylméthoxy)carbonyl]amino]éthyl]phosphinique synthétisé en 1.4., 2,2 g (14,10 mmol) de 4-méthyl-2-méthylènepentanoate d'éthyle et 0,52 ml de *N,O*-bis(triméthylsilyl) acétamide dans 0,27 ml d'acétonitrile est agitée à une température voisine de 20 °C pendant 24 heures. On ajoute 60 ml d'un mélange acétate d'éthyle / eau : 1 / 1. On extrait la phase aqueuse avec de l'acétate d'éthyle, l'ensemble des phases organiques est lavé à l'eau, séché sur sulfate de sodium, filtré et évaporé sous vide. On purifie par recristallisation dans un mélange d'acétate d'éthyle et d'hexane.
On récupère 1,21 g de produit (rendement = 81,3%).
Point de fusion : 143-144°C.

4.4. Acide 2-[[Hydroxy[2-phényl-1-[[(phénylméthoxy)carbonyl] amino]éthyl]phosphinyl]méthyl]-9-méthylpentanoïque

On procède selon les conditions opératoires décrites en 1.7. à partir de 510 mg (1,07 mmol) de 2-[[Hydroxy [2-phényl-1-[[(phénylméthoxy)carbonyl]amino]éthyl]phosphinyl] méthyl]-4-méthylpentanoate d'éthyle.
On récupère 479 mg de produit (rendement = 99,8%).
Point de fusion : 170-172°C.

4.5. *N*-[2-[[Hydroxy[2-phényl-1-[[(phénylméthoxy) carbonyl]amino]éthyl]phosphinyl]méthyl]-4-méthyl-1-oxopentyl] -*L*-phénylalaninate de méthyle

On procède selon le mode opératoire décrit en 1.8. à partir de 250 mg (0,58 mmol) de l'acide 2-[[Hydroxy [2-phényl-1-[[(phénylméthoxy)carbonyl]amino]éthyl]phosphinyl]méthyl]-4-méthylpentanoïque et 124,5 mg (0,58 mmol) de chlorhydrate de phénylalaninate de méthyle.
On récupère 228 mg de produit (rendement = 65%).
Point de fusion : 150-152°C.

4.6. *N*-[2-[[Hydroxy[2-phényl-1-[[(phènylméthoxy)carbonyl] amino]éthyl]phosphinyl]méthyl]-4-méthyl-1-oxopentyl]-*L*-phénylalanine

On procède selon le mode opératoire décrit en 1.7. à partir de 180 mg (0,30 mmol) de *N*-[2-[[Hydroxy[2-phényl-1-[[(phénylméthoxy)carbonyl]amino]éthyl]phosphinyl]méthyl]-4-méthyl-1-oxopentyl]-*L*-phénylalaninate de méthyle.
On récupère 158 mg de produit (rendement = 89,9%).
Point de fusion : 171-173°C.

4.7. Bromhydrate de *N*-[2-[[(1-amino-2-phényléthyl) hydroxyphosphinyl]méthyl]-4-méthyl-1-oxopentyl]-*L*-phénylalanine

On procède selon le mode opératoire décrit en 2.3. à partir de 120 mg (0,20 mmol) de *N*-[2-[[Hydroxy[2-phényl-1-[[(phényl_ méthoxy)carbonyl]amino]éthyl]phosphinyl]méthyl]-4-méthyl-1-oxopentyl]-*L*-phénylalanine.
On récupère 109 mg de produit (rendement = 100%).

Point de fusion : 230°C (décomposition).

Exemple 5 : Bromhydrate de *N*-[3-[(1-amino-2-phényl éthyl)hydroxyphosphinyl]-2-([1,1'-biphényl]-4-ylméthyl)-1-oxopropyl]-*L*-alanine

5.1. 4-bromo-α-(diéthoxyphosphinyl)benzènepropanoate d'éthyle

A partir de 9 ml (45,36 mmol) de triéthylphosphonoacétate et 11,8 g (47,34 mmol) de bromure de (4-bromophényl)méthyle, on procède selon les conditions opératoire décrites en 4.1.
On récupère 7,8 g de produit sous forme d'huile
(rendement = 43,73%).
Rf (heptane / acétate d'éthyle : 1 / 4) : 0,46.

5.2. 4-Bromo-α-méthylènebenzènepropanoate d'éthyle

a partir de 5,5 g (14 mmol) de 4-bromo-α-(diéthoxyphosphinyl) benzènepropanoate d'éthyle et 5,3 ml (71 mmol) de formaldéhyde. On procède selon les conditions opératoires décrites en 4.2. On purifie par chromatographie sur gel de silice en éluant avec un mélange acétate d'éthyle / heptane : 1 / 20.
On récupère 2,82 g de produit sous forme d'huile
(rendement = 75%).
Rf (heptane / acétate d'éthyle : 9 / 1) : 0,57.

5.3. 3-(4-bromophényl)-2-[[hydroxy[2-phényl-1-[[(phényl_ méthoxy)carbonyl]amino]éthyl]phosphinyl]méthyl]propanoate d'éthyle

On procède selon les conditions opératoires décrites en 1.6. à partir de 2 g (6,27 mmol) de l'acide [2-phényl-1-[(phénylméthoxy)carbonyl]aminoéthyl]phosphinique synthétisé en 1.4. et 2 g (7,44 mmol) de 4-Bromo-α-méthylènebenzène propanoate d'éthyle.
On récupère 3,1 g de produit (rendement = 84,1%).
Point de fusion : 124-125°C.

5.4. 3-[1,1'-biphényl]-4-yl-2-[[hydroxy[2-phényl-1-[[(phénylméthoxy)carbonyl]amino]éthyl]phosphinyl]méthyl] propanoate d'éthyle

On condense 200 mg (0,34 mmol) de 3-(4-bromophényl)-2-[[hydroxy[2-phényl-1-[[(phénylméthoxy)carbonyl] amino]éthyl] phosphinyl]méthyl]propanoate d'éthyle avec 42 mg (0,34 mmol) de l'acide phénylboronique en opérant dans 1,8 ml d'un mélange toluène / méthanol : 2 / 1, de tétrakis(triphénylphosphine)palladium et de 0,4 ml de carbonate de sodium 2M (0,8 mmol). Le mélange réactionnel est agité pendant 6 heures sous azote et à reflux. Après refroidissement, on ajoute 10 ml d'acétate d'éthyle et acidifie jusqu'à pH = 3 par une solution aqueuse d'acide chlorhydrique 2N. Après filtration sur célite, rincé par de l'acétate, le solvant est évaporé sous pression réduite. On purifie par recristallisation dans un mélange d'acétate d'éthyle et d'hexane.
On récupère 180 mg de produit (rendement = 90,4%).
Point de fusion : 164-165°C.

5.5. Acide 3-[1,1'-biphényl]-4-yl-2-[[hydroxy[2-phényl-1-[[(phénylméthoxy)carbonyl]amino]éthyl]méthyl] phosphinyl] propanoïque

On procède selon les conditions opératoires décrites en 1.7. à partir de 180 mg (0,31 mmol) de 3-[1,1'-biphényl]-4-yl-2-[[hydroxy[2-phényl-1-[[(phénylméthoxy)carbonyl]amino]éthyl] phosphinyl]méthyl]propanoate d'éthyle.
On récupère 167 mg de produit (rendement = 97,4%).
Point de fusion : 180-182°C.

5.6. *N*-[3-[1,1'-biphényl]-4-yl-2-[[hydroxy[2-phényl-1-[[(phénylméthoxy)carbonyl]amino]éthyl]phosphinyl]méthyl]-1-oxopropyl]-*L*-alaninate de méthyle

On procède selon les conditions opératoires décrites en 1.8 à partir de 160 mg (0,29 mmol)de l'acide 3-[1,1'-biphényl]-4-yl-2-[[hydroxy[2-phényl-1-[[(phénylméthoxy)carbonyl]amino] éthyl]méthyl]phosphinyl]propanoïque et

124,5 mg (0,58 mmol) de chlorhydrate d'alaninate de méthyle.
On récupère 120 mg de produit (rendement = 65%).
Point de fusion : 168-171°C.

5.7. *N*-[3-[1,1'-biphényl]-4-yl-2-[[hydroxy[2-phényl-1-[[(phénylméthoxy)carbonyl]amino]éthyl]phosphinyl]méthyl]-1-oxopropyl]-*L*-alanine

On procède selon les conditions opératoires décrites en 1.7. à partir de 94 mg (0,30 mmol) de *N*-[3-[1,1'-biphényl]-4-yl-2-[[hydroxy[2-phényl-1-[[(phénylméthoxy)carbonyl]amino]éthyl] phosphinyl]méthyl]-1-oxopropyl]-*L*-alaninate de méthyle.
On récupère 92 mg de produit (rendement = 100%).
Point de fusion : 189-192°C.

5.8. Bromhydrate de *N*-[3-[(1-amino-2-phényl_ éthyl)hydroxyphosphinyl]-2-([1,1'-biphényl]-4-ylméthyl)-1-oxopropyl] -*L*-alanine

On procède selon les conditions opératoires décrites en 2.3 à partir de 90 mg (0,14 mmol) de *N*-[3-[1,1'-biphényl]-4-yl-2-[[hydroxy[2-phényl-1-[[(phénylméthoxy)carbonyl]amino]éthyl] phosphinyl]méthyl]-1-oxopropyl]-*L*-alanine.
On récupère 82,4 mg (rendement = 100%).
Point de fusion : 210°C (décomposition).
*<u>Sont obtenus de manière analogue</u> :*

Les composés n°22, 23, 24 du tableau.

<u>Exemple 6</u> : <u>Bromhydrate de *N*-[2-[[(1-aminoéthyl)hydroxy phosphinyl]méthyl]-1-oxo-3-phénylpropyl]-*L*-phénylalanine</u>

6.1. Acide [1-[(diphénylméthyl)amino]éthyl]phosphinique

Une solution de 10 g (45,45 mmol) de chlorhydrate de diphénylméthylamine et 4,8 ml (46,33 mmol) de l'acide hypophosphoneux à 50% dans l'eau dans 100 ml d'un mélange eau / éthanol : 90 / 10, est portée au reflux. On ajoute goutte à goutte, 4 g (0,09 mmol) d'acétaldéhyde. Après addition, on maintient le reflux pendant une heure. Le précipité obtenu est filtré et lavé avec 100 ml d'eau et 100 ml d'acétone.
On récupère 8 g de produit (rendement = 64%).
Point de fusion : 236°C.

6.2. Acide (1-aminoéthyl)phosphinique

Un mélange de 4,5 g (16,36 mmol) d'acide [1-[(diphénylméthyl) amino]éthyl]phosphinique et de 30 ml d'acide chlorhydrique 6N est porté à reflux pendant 2 heures. Deux phases apparaissent. Le mélange est mis à sec par évaporation sous vide à chaud puis repris dans 30 ml d'eau. On lave trois fois la phase aqueuse au diéthyléther. La phase aqueuse, à nouveau évaporée, conduit au chlorhydrate de l'acide (1-aminoéthyl)phosphinique, qui est repris dans 15 ml d'éthanol absolu. On ajoute 8 ml d'oxyde de propylène, goutte à goutte, sous agitation, à 0°C. On observe la formation d'un précipité blanc abondant qui est filtré et lavé au diéthyléther
On récupère 1,7 g de produit (rendement = 95,3%).
Point de fusion : 222-224°C (décomposition).

6.3. Acide [1-[[(phénylméthoxy)carbonyl]amino]éthyl] phosphinique

On procède selon les conditions opératoires décrites en 1.4. à partir de 1,7 g (15,6 mmol) d'acide (1-aminoéthyl) phosphinique et de 3,12 g (18,29 mmol) de chloroformiate de benzyle.
On récupère 2,8 g de produit (rendement = 73,9%).
Point de fusion : 132-134°C.

6.4. $\alpha$-[[Hydroxy[1-[[(phénylméthoxy)carbonyl]amino]éthyl] phosphinyl]méthyl]benzènepropanoate de méthyle

Une solution de 0,6 g (2,47 mmol) de l'acide [1-[[(phénylméthoxy)carbonyl]amino]éthyl]phosphinique, 2,17 g (12,3 mmol) de 2-(phénylméthyl)propénoate de méthyle et 0,52 ml de *N*,*O*-bis(triméthylsilyl)acétamide dans 0,27 ml

d'acétonitrile est agitée à une température voisine de 20°C pendant 24 heures. On ajoute 60 ml d'un mélange acétate d'éthyle / eau : 1 / 1. On extrait la phase aqueuse par de l'acétate d'éthyle. L'ensemble des phases organiques est lavé à l'eau, séché sur sulfate de sodium, filtré et évaporé sous vide. On purifie par recristallisation dans un mélange d'acétate d'éthyle et d'hexane.
On récupère 0,71 g de produit (rendement = 68,9%).
Point de fusion : 154-155°C.

6.5. Bromhydrate de *N*-[2-[[(1-aminoéthyl)hydroxyphosphinyl] méthyl]-1-oxo-3-phénylpropyl]-*L*-phénylalanine

On procède successivement, en utilisant à chaque fois comme produit de départ le composé synthétisé à l'étape précédente, selon les conditions opératoires décrites en 1.7. (rendement = 90,2%), en 1.8. (rendement = 89,4%), en 1.7. (rendement = 91%) puis en 2.3. (rendement = 100%).
Le carboxylate d'α-aminoacide utilisé est le phénylalaninate de méthyle.
Point de fusion : 230°C (décomposition).

Exemple 7 : Bromhydrate de *N*-[3-[(1-aminoéthyl)hydroxy phosphinyl]-2-([1,1'-biphényl)-4-ylméthyl)-1-oxopropyl]-*L*-phénylalanine

7.1. 3-(4-bromophényl)-2-[[hydroxy[1-[[(phénylméthoxy) carbonyl]amino]éthyl]phosphinyl]méthyl]propanoate d'éthyle

On procède selon les conditions opératoires décrites en 5.3. à partir de 2,6 g (9,67 mmol) de 4-bromo-α-méthylènebenzène_ propanoate d'éthyle et 2 g (8,23 mmol) de l'acide [1-[[(phénylméthoxy)carbonyl]amino]éthyl]phosphinique.
On récupère 3,79 g de produit (rendement = 90%).
Point de fusion : 120-122°C.

7.2. 3-[1,1'-biphényl]-4-yl-2-[[hydroxy[1-[[(phénylméthoxy) carbonyl]amino]éthyl]phosphinyl]méthyl]propanoate d'éthyle

On procède selon les conditions opératoires de 5.4 à partir de 1,7 g (3,32 mmol) de 3-(4-bromophényl)-2-[[hydroxy[1-[[(phénylméthoxy)carbonyl]amino]éthyl]phosphinyl]méthyl] propanoate d'éthyle et 4,05 g (3,32 mmol) de l'acide phénylboronique.
On récupère 1,65 g de produit (rendement = 97,6%).
Point de fusion : 198°C (décomposition).

7.3. Bromhydrate de *N*-[3-[(1-aminoéthyl)hydroxy_ phosphinyl]-2-([1,1'-biphényl]-4-ylméthyl)-1-oxopropyl]-*L*-phénylalanine

On procède successivement en utilisant à chaque fois comme produit de départ le composé synthétisé à l'étape précédente selon les conditions opératoires décrites en 1.7. (rendement = 88,2%), en 1.8. (rendement = 65%), en 1.7. (rendement = 95%) puis en 2.3. (rendement = 100%).
Point de fusion : 225°C (décomposition).

Exemple 8 : Bromhydrate de *N*-[3-[(1-aminoéthyl)hydroxy phosphinyl]-2-([1,1'-biphényl]-4-ylméthyl)-1-oxopropyl]-*L*-alanine

On procède selon les conditions opératoires décrites en 5.3. à partir de 4-bromo-α-méthylènebenzènepropanoate d'éthyle et d'acide [1-[[(phénylméthoxy)carbonyl]amino]éthyl] phosphinique, en 5.4. à partir du composé obtenu puis, On procède successivement, en utilisant à chaque fois comme produit de départ le composé synthétisé à l'étape précédente, selon les conditions opératoires décrites en 1.7. (rendement = 88,2%), en 1.8. (rendement = 83%), en 1.7. (rendement = 95%) puis en 2.3. (rendement = 100%).
Point de fusion : 225°C (décomposition).

Exemple 9 : Bromhydrate de *N*-[2-[[(aminophénylméthyl)hydroxy phosphinyl]méthyl]-1-oxo-3-phénylpropyl]-*L*-phénylalanine

9.1. Acide [1-(diphénylméthyl)amino-2-phénylméthyl] phosphinique

On procède selon les conditions opératoires décrites en 1.2. à partir de 5 g (20 mmol) de phosphinate de α-phénylbenzèneméthanamine et de 4,08 g (40,12 mmol) de benzaldéhyde.
On récupère 2,9 g de produit (rendement = 43%).
Point de fusion ; 212°C.

9.2. Acide (aminophénylméthyl)phosphinique

On procède selon les conditions opératoires décrites en 1.3. à partir de 22,1 g (65,58 mmol) d'acide [1-(diphénylméthyl) amino-2-phénylméthyl]phosphinique.
On récupère 9,6 g de produit (rendement = 86%).
Point de fusion : 243°C.

9.3. Acide [[[(phénylméthoxy)carbonyl]amino]phényl_ méthyl]phosphinique

On procède selon les conditions opératoires décrites en 1.4. à partir de 1 g (5,85 mmol) d'acide (aminophénylméthyl) phosphinique et 1,17 g (6,86 mmol) de chloroformiate de benzyle.
On récupère 1,6 g de produit (rendement = 90%).
Point de fusion : 143°C.

9.4. Bromhydrate de *N*-[2-[[(aminophénylméthyl)hydroxy_ phosphinyl]méthyl]-1-oxo-3-phénylpropyl]-*L*-phénylalanine

On procède selon les conditions opératoires décrites en 1.6. à partir de 2-(phénylméthyl)propénoate de méthyle et d'acide [[[(phénylméthoxy)carbonyl]amino]phénylméthyl]phosphinique (rendement = 64%), puis, On procède successivement, en utilisant à chaque fois comme produit de départ le composé synthétisé à l'étape précédente, selon les conditions opératoires décrites en 1.7. (rendement = 85%), en 1.8. (rendement = 61%), en 1.7. (rendement = 98%) puis en 2.3. (rendement = 100%).
Point de fusion : 165°C (décomposition).

Exemple 10 : Bromhydrate de *N*-[3-[(aminophénylméthyl)hydroxy phosphinyl]-2-([1,1'-biphényl]-4-ylméthyl)-1-oxopropyl]-*L*-alanine

On procède selon les conditions opératoires décrites en 1.6. à partir de l'acide[[[(phénylméthoxy)carbonyl] amino] phénylméthyl]phosphinique et de 2-[(4-Bromophényl)méthyl] propénoate d' éthyle (rendement = 87%), puis on procède successivement en utilisant à chaque fois comme produit de départ le composé synthétisé à l'étape précédente, selon les conditions opératoires décrites en 5.4. (rendement = 84%), en 1.7. (rendement = 99%), en 1.8. (rendement = 60%), en 1.7. (rendement = 95%), puis en 2.3. (rendement = 80%).
Point de fusion : 194°C (décomposition).

Exemple 11 : Trifluoroacétate de *N*-[2-[[(1-amino-2-phényléthyl)hydroxyphosphinyl]méthyl]-1-oxo-3-phénylpropyl]-*L*-phénylalaninate de phénylméthyle

11.1. Acide 3-[hydroxy[2-phényl-1-[[(*tert*-butoxy)carbonyl] amino]éthyl]phosphinyl]-2-(phénylméthyl)propanoique

L'acide 3-[hydroxy[2-phényl-1-[[(phénylméthoxy)carbonyl] amino]éthyl]phosphinyl]-2-(phénylméthyl)propanoïque est traité par du tribromure de bore selon les conditions opératoires décrites en 2.3. Le bromhydrate obtenu est isolé puis traité par du di-*tert*-butyl dicarbonate en présence de triéthylamine dans un solvant organique tel que le *N*,*N*-diméthylformamide. On ajoute de l'eau, on extrait par l'acétate d'éthyle. Les phases organiques sont lavées à l'eau, séchées sur sulfate de sodium.
On récupère le produit avec un rendement de 61%.

11.2. *N*-[2-[[Hydroxy[2-phényl-1-[[(*tert*-butoxy)carbonyl] amino]éthyl]phosphinyl]méthyl]-1-oxo-3-phénylpropyl]-*L*-phénylalaninate de phénylméthyle

L'acide 3-[hydroxy[2-phényl-1-[[(*tert*-butoxy)carbonyl] amino]éthyl]phosphinyl]-2-(phénylméthyl)propanoïque est traité selon les conditions opératoires de 1.8.
On récupère le produit avec un rendement de 72%.

11.3. Trifluoroacétate de *N*-[2-[[(1-amino-2-phényléthyl)hydroxyphosphinyl]méthyl]-1-oxo-3-phénylpropyl]-*L*-phénylalaninate de phénylméthyle

Le *N*-[2-[[hydroxy[2-phényl-1-[[(*tert*-butoxy)carbonyl] amino]éthyl]phosphinyl]méthyl]-1-oxo-3-phénylpropyl] -*L*-phénylalaninate de phénylméthyle est traité par le mélange acide trifluoroacétique / dichlorométhane, une heure, à température ambiante. On évapore à sec. Le produit obtenu est précipité dans l'ether.
On récupère le produit avec un rendement de 98%.
Point de fusion : 210°C (décomposition).

<u>Exemple 12</u> : <u>Formiate de *N*-[2-[[(1-amino-2-phényléthyl) phénylméthoxyphosphinyl]méthyl]-1-oxo-3-phénylpropyl]-L-phénylalaninate de phénylméthyle</u>

12.1. *N*-[2-[[(2-Phényl-1-[[(tert-butoxy)carbonyl] amino]éthyl]phénylméthoxyphosphinyl]méthyl]-1-oxo-3-phénylpropyl]-*L*-phénylalaninate de phénylméthyle

Le *N*-[2-[[hydroxy[2-phényl-1-[[(*tert*-butoxy)carbonyl] amino]éthyl]phosphinyl]méthyl]-1-oxo-3-phénylpropyl] -*L*-phénylalaninate de phénylméthyle est traité par l'alcool benzylique (1,1 éq.) en présence de dicyclohexylcarbodiimide et de diméthylaminopyridine dans le tétrahydrofurane. Après agitation pendant une nuit à température ambiante, on filtre puis évapore à sec. Le résidu est repris par de l'acétate d'éthyle. On lave 3 fois à l'eau, on sèche sur sulfate de sodium puis on évapore à sec. On purifie par chromatographie sur gel de silice en éluant par un mélange dichlorométhane /méthanol : 1 / 9.
On récupère le produit avec un rendement de 60%.

12.2. Formiate de *N*-[2-[[(1-amino-2-phényléthyl) phénylméthoxyphosphinyl]méthyl]-1-oxo-3-phénylpropyl]-*L*-phénylalaninate de phénylméthyle

Le *N*-[2-[[[2-phényl-1-[[(*tert*-butoxy)carbonyl] amino]éthyl]phénylméthoxyphosphinyl]méthyl]-1-oxo-3-phénylpropyl]-*L*-phénylalaninate de phénylméthyle est agité pendant 2 heures en présence d'acide formique. On évapore à sec. On précipite par du diéthyléther.
On récupère le produit avec un rendement de 97%.
Point de fusion : 192°C (décomposition).

<u>Exemple 13</u> : <u>Trifluoroacétate de N-[3-[(1-aminoéthyl) hydroxyphosphinyl]-2-([1,1'-biphényl]-4-ylméthyl)-1-oxopropyl] -*L*-alaninate de phénylméthyle</u>

On procède selon les conditions opératoires décrites en 11.1 à partir de l'acide 3-[1,1'-biphényl]-4-yl-2-[[hydroxy [2-phényl-1-[[(phénylméthoxy)carbonyl]amino]éthyl]méthyl] phosphinyl]propanoïque. Le composé obtenu est traité par le L-alaninate de phénylméthyle selon les conditions opératoires décrites en 11.2. (rendement = 61%) puis en 11.3. (rendement = 96%).
Point de fusion : 205°C (décomposition).

Les autres composés du tableau sont synthétisés de façon analogue à partir des produits de départ adéquats.

Le tableau suivant rassemble les composés de l'invention, ainsi que leurs caractéristiques physiques.

Lorque n est égal à un dans ce tableau, $R_7$ représente un atome d'hydrogène.

<u>TABLEAU</u>

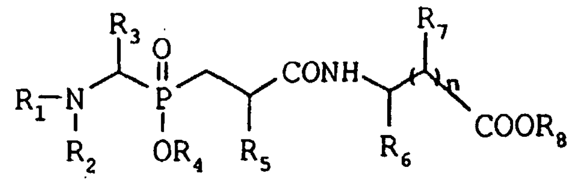

(I)

| N° | $R_1$ | $R_2$ | $R_3$ | $R_4$ | $R_5$ | $R_6$ | $R_8$ | n | P.F. (°C) | Tr (min) % acéto-nitrile |
|---|---|---|---|---|---|---|---|---|---|---|
| 1 | H | H | 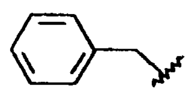 | H | 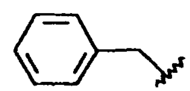 | 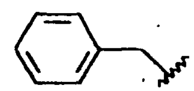 | H | 0 | 220 (HCl) | A : 12,01<br>B : 12,8<br>C : 14,7<br>D : 22,0<br>30% |
| 2 | H | H | 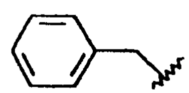 | H | 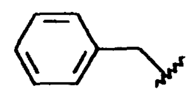 | $-CH_3$ | H | 0 | 210 (HBr) | A+B : 5,4<br>C+D : 8,8<br>30% |
| 3 | H | H | 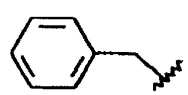 | H | 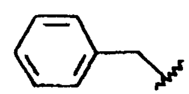 | $-CH_2CH(CH_3)_2$ | H | 0 | 230 (HBr) | A+B : 3,3<br>C+D : 7,2<br>35% |

| N° | R1 | R2 | R3 | R4 | R5 | R6 | R8 | n | P.F. | Tr |
|---|---|---|---|---|---|---|---|---|---|---|
| 4 | H | H | 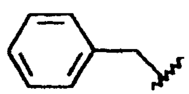 | H | 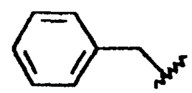 | 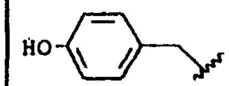 | H | 0 | 160 (HBr) | A+B : 8,9<br>C : 12,0<br>D : 14,3<br>25% |
| 5 | H | H | 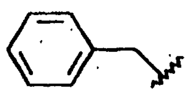 | H | 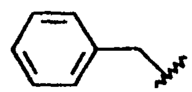 | 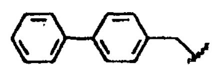 | H | 0 | 170 (HBr) | A+B : 7,1<br>C : 8,8<br>D : 9,2<br>43% |
| 6 | H | H | 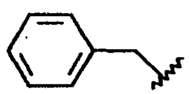 | H | 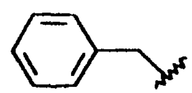 | 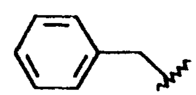 | H | 1 | 240 (HBr) | A : 9,7<br>B : 10,3<br>C : 20,9<br>D : 25,6<br>30% |
| 7 | H | H | $-CH_3$ | H | 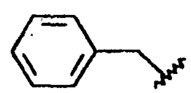 | 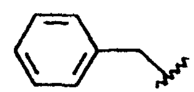 | H | 0 | 230 (HBr) | A+B : 5,4<br>C+D : 8,8<br>30% |

| N° | $R_1$ | $R_2$ | $R_3$ | $R_4$ | $R_5$ | $R_6$ | $R_8$ | n | P.F. | Tr |
|---|---|---|---|---|---|---|---|---|---|---|
| 8 | H | H | $-CH_3$ | H | 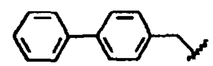 | 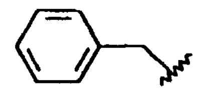 | H | 0 | 175 (HBr) | A+B : 8,5 C+D : 10,9 35% |
| 9 | H | H | $-CH_3$ | H | 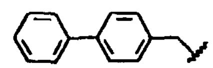 | $-CH_3$ | H | 0 | 225 (HBr) | A+B : 7,2 C+D : 10,3 30% |
| 10 | H | H | $-CH_2CH_3$ | H | 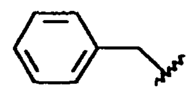 | 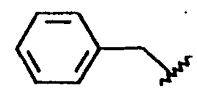 | H | 0 | 204 (HBr) | A+B : 6,2 C+D : 10,4 28% |
| 11 | H | H | $-CH_2CH(CH_3)_2$ | H | 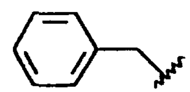 | 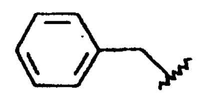 | H | 0 | 195 (HBr) | A+B : 5,3 C+D : 7,2 35% |

| N° | $R_1$ | $R_2$ | $R_3$ | $R_4$ | $R_5$ | $R_6$ | $R_8$ | n | P.F. | Tr |
|---|---|---|---|---|---|---|---|---|---|---|
| 12 | H | H | $-CH_2CH_2SCH_3$ | H | 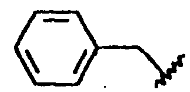 | 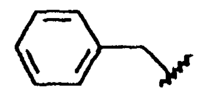 | H | 0 | 175 (HBr) | A+B : 6,5<br>C+D : 10,4<br>30% |
| 13 | H | H | 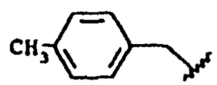 | H | 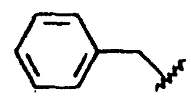 | 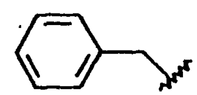 | H | 0 | 165 (HBr) | A : 10,1<br>B : 10,5<br>C+D : 12,7<br>35% |
| 14 | H | H | 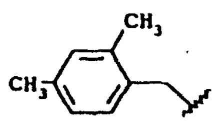 | H | 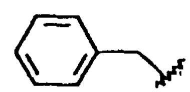 | 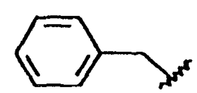 | H | 0 | 185 (HBr) | A+B : 16,7<br>C : 28,8<br>D : 31,9<br>30% |
| 15 | H | H | 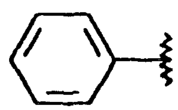 | H | 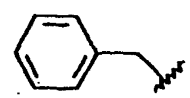 | 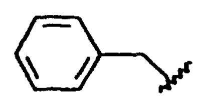 | H | 0 | 173 (HBr) | A : 8,25<br>B : 8,7<br>C+D : 14,0<br>30% |

| N° | $R_1$ | $R_2$ | $R_3$ | $R_4$ | $R_5$ | $R_6$ | $R_8$ | n | P.F. | Tr |
|---|---|---|---|---|---|---|---|---|---|---|
| 16 | H | H | 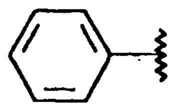 | H | 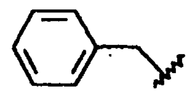 | 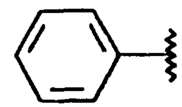 | H | 0 | 190 (HBr) | A+B : 3,7<br>C+D : 9,4<br>30% |
| 17 | H | H | 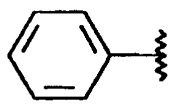 | H | $-CH_2CH(CH_3)_2$ | 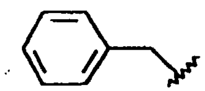 | H | 0 | 150 (HBr) | A : 8,0<br>B : 8,7<br>C+D : 12,5<br>30% |
| 18 | H | H | 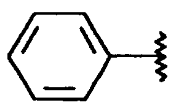 | H | 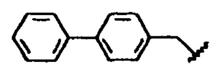 | $-CH_3$ | H | 0 | 194 (HBr) | A+B : 7,5<br>C+D : 10,4<br>33% |
| 19 | H | H | 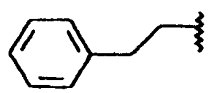 | H | 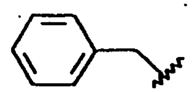 | 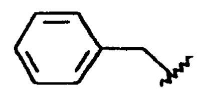 | H | 0 | 205 (HBr) | A+B : 5,1<br>C : 5,4<br>D : 7,0<br>30% |

| N° | $R_1$ | $R_2$ | $R_3$ | $R_4$ | $R_5$ | $R_6$ | $R_8$ | n | P.F. | Tr |
|---|---|---|---|---|---|---|---|---|---|---|
| 20 | H | H | 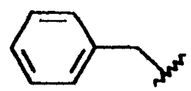 | H | $-CH_2CH(CH_3)_2$ | 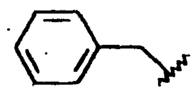 | H | 0 | 230 (HBr) | A : 8,5<br>B : 10,1<br>C : 14,3<br>D : 15,1<br>30% |
| 21 | H | H | 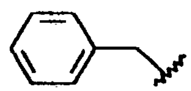 | H | 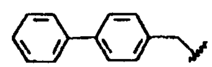 | $-CH_3$ | H | 0 | 210 (HBr) | A+B : 15,7<br>C : 21,3<br>D : 23,2<br>30% |
| 22 | H | H | 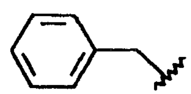 | H | 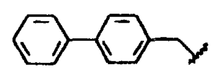 | $-CH_2CH_3$ | H | 0 | 205 (HBr) | A+B : 10,0<br>C : 14,4<br>D : 15,5<br>30% |
| 23 | H | H | 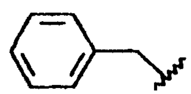 | H | 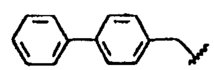 | $-CH_2CH(CH_3)_2$ | H | 0 | 221 (HBr) | A+B : 11,4<br>C :14,0<br>D : 14,9<br>30% |

| N° | $R_1$ | $R_2$ | $R_3$ | $R_4$ | $R_5$ | $R_6$ | $R_8$ | n | P.F. | Tr |
|---|---|---|---|---|---|---|---|---|---|---|
| 24 | H | H | 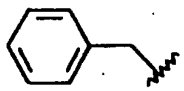 | H | 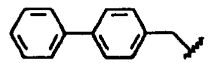 | 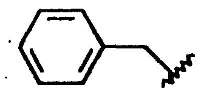 | H | 0 | 225 (HBr) | A+B : 8,7<br>C : 10,3<br>D : 10,8<br>45% |
| 25 | H | H | $-CH_3$ | H | 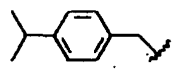 | $-CH_3$ | H | 0 | 140 (HBr) | A+B : 7,8<br>C+D : 12,6<br>27% |
| 26 | H | H | $-CH(CH_3)$ | H | 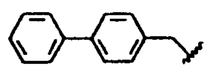 | $-CH_3$ | H | 0 | 196 (HBr) | A+B : 9,2<br>C+D : 12,5<br>30% |
| 27 | H | H | 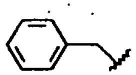 | H | 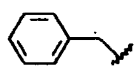 | 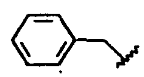 | 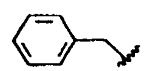 | 0 | 210* | A+B : 6,4<br>C : 7,3<br>D : 7,8<br>50% |

| N° | $R_1$ | $R_2$ | $R_3$ | $R_4$ | $R_5$ | $R_6$ | $R_8$ | n | P.F. | Tr |
|---|---|---|---|---|---|---|---|---|---|---|
| 28 | H | H | 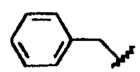 | 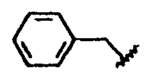 | 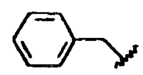 | 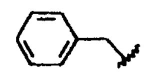 | 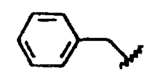 | 0 | 192** | A+B : 18,5<br>C+D : 21,2<br>50% |
| 29 | H | H | $-CH_3$ | H | 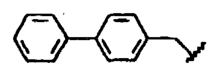 | $-CH_3$ | 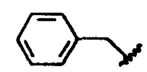 | 0 | 205* | B : 6,0<br>50% |

- Tous ces composés se décomposent à la température de leur point de fusion

- Les temps de rétention ont été mesuré par HPLC sur colonne Chromasil (5 µM, 100 Å, diamètre : 4,6 mm, hauteur : 250 mm), l'éluant utilisé est un mélange Acétonitrile / eau (0,05% d'acide trifluoroacétique). Le pourcentage d'acétonitrile étant donné dans le tableau

* : (trifluoroacétate)

** . (formiate)

Les composés de l'invention ont fait l'objet d'essais enzymologiques permettant de déterminer leur pouvoir inhibiteur de l'EPN et de l'APN.

Mesure du pouvoir inhibiteur sur l'endopeptidase neutre (EPN)

Le pouvoir inhibiteur est déterminé sur de l'endopeptidase neutre purifiée de rein de lapin en suivant le protocole décrit dans la littérature (Llorens et al., *Neurochem.*, **39,** 1081, 1982). Après une incubation de 15 minutes à 25 °C, un aliquot de protéines est incubé pendant 20 minutes à 37°C en présence de 20 nmoles de ($^3$H)D-Ala$^2$-Leu$^5$-enképhaline et du composé à tester en solution dans le tampon Tris HCl (pH = 7,4).
On arrête la réaction par addition d'acide chlorhydrique 0,2 N. Le métabolite tritié ($^3$H)-Tyr-D-Ala-Gly est séparé de la D-Ala$^2$-Leu$^5$-enképhaline par chromatographie sur colonne de Porapak et la quantité de métabolite formée mesurée à l'aide d'un compteur à scintillation liquide.
L'activité des différents composés de l'invention, exprimée en concentrations inhibitrices 50 ($CI_{50}$) varie de $10^{-6}$ à $10^{-9}$ M.

Pouvoir inhibiteur sur l'aminopeptidase N (APN)

Le pouvoir inhibiteur est mesuré sur l'aminopeptidase purifiée du rein de porc (Boehringer, France). Après une préincubation de 15 minutes à 25°C, un aliquot de protéines est incubé pendant 20 minutes à 25 °C en présence de 20 nmoles de ($^3$H)-Leu-enképhaline et du composé à tester en solution dans un tampon tris Hcl (pH = 7,4).
On arrête la réaction par addition d'acide chlorhydrique 0,5 N. Le métabolite formé ($^3$H)Tyr est séparé par chromatographie sur colonne de Porapak et la quantité de métabolite formée, mesurée à l'aide d'un compteur à scintillation liquide.
L'activité des différents composés de l'invention, exprimée en concentrations inhibitrices 50 ($CI_{50}$) varie de $10^{-6}$ à $10^{-9}$ M.
Les composés de l'invention ont également fait l'objet d'essais pharmacologiques permettant de mesurer leur activité analgésique.

Test de la plaque chaude chez la souris

Le test est effectué 15 minutes après administration par voie intracérébroventriculaire de doses croissantes des composés de l'invention chez la souris.
On mesure deux paramètres : le temps de latence du saut et la latence du léchage.
Les résultats, exprimés en $DE_{50}$, c'est-à-dire en doses donnant la moitié de la réponse maximale. L'activité analgésique des différents composés de l'invention se situent entre 1 et 100 µg/kg. L'activité des composés de l'invention les plus actifs se situent entre 1 à 20 µg/kg.
Les composés de l'invention présentent une activité mixte EPN/APN in vitro et une activité analgésique in vivo ; ces résultats montrent que les composés de l'invention peuvent être utilisés comme analgésiques.
Les composés selon l'invention peuvent aussi être mis en oeuvre pour la préparation de médicaments destinés au traitement des états dépressifs de toute nature, des troubles du sommeil, des troubles de l'anxiété, des troubles cognitifs et de la vigilance, et des troubles d'ordre périphériques (diarrhée, toux, hypertension, inflammation...).
L'invention a également pour objet des compositions pharmaceutiques comprenant à titre de principe actif l'un au moins des composés de formule (I) ou leurs sels d'addition en association avec tout excipient approprié.
Les composés de l'invention peuvent être présentés, en association avec des excipients, sous forme de compositions formulées en vue de l'administration par voie entérale ou parentérale, par exemple sous forme de comprimés, de pilules, de granulés, de poudres, de dragées, de capsules, de solutions, de suspensions, de solutions injectables, d'élixirs ou de sirops.
Les solutions des sels des composés de l'invention sont particulièrement utiles pour l'administration par injection intramusculaire ou sous-cutanée.
Les composés de l'invention sont administrés à une dose journalière comprise entre 0,01 et 100 mg/kg, de préférence entre 0,1 et 10 mg/kg.

annexe 1

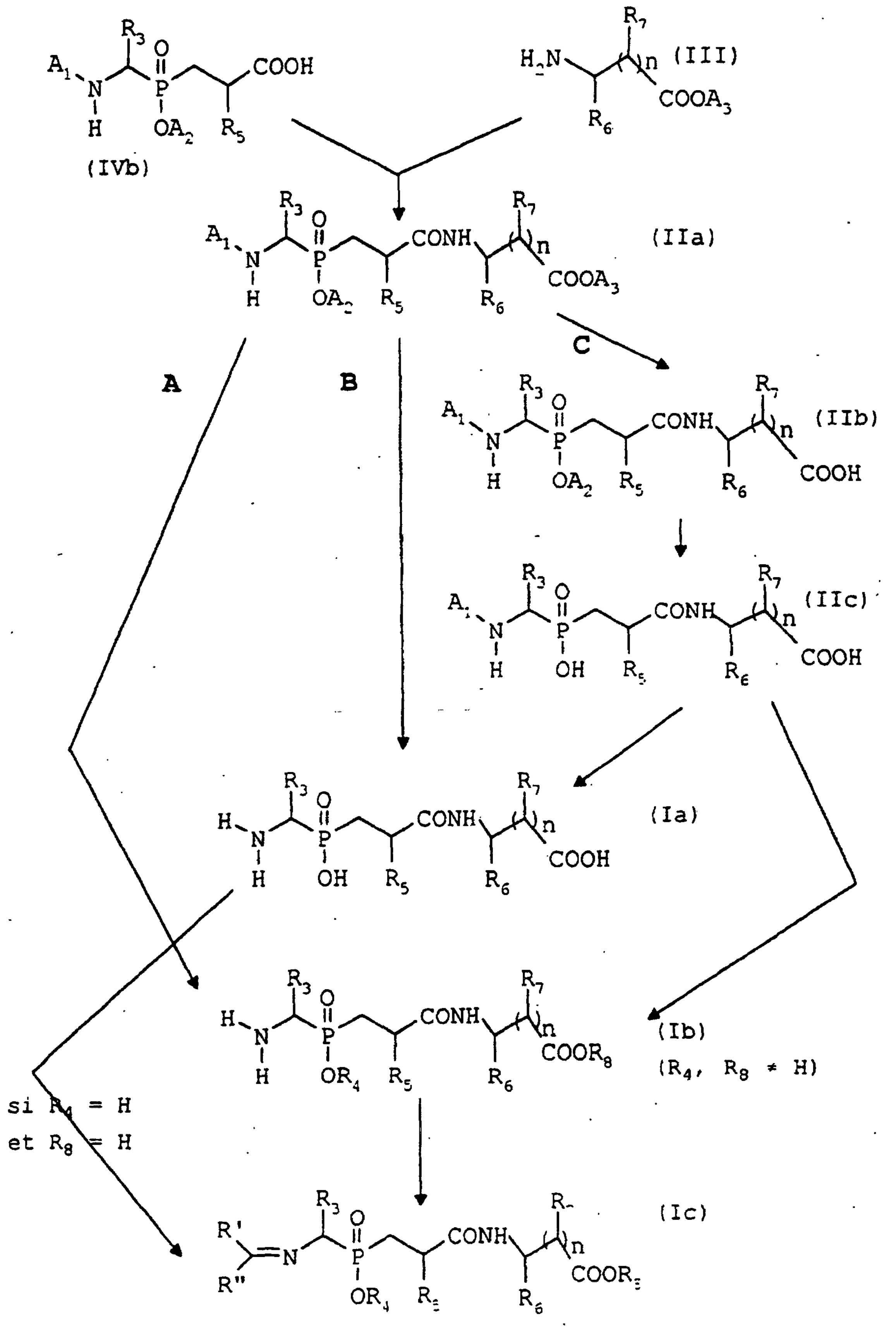


(IVb) (III) (IIa) (IIb) (IIc) (Ia) (Ib) (Ic)

annexe 2

*schéma 1*

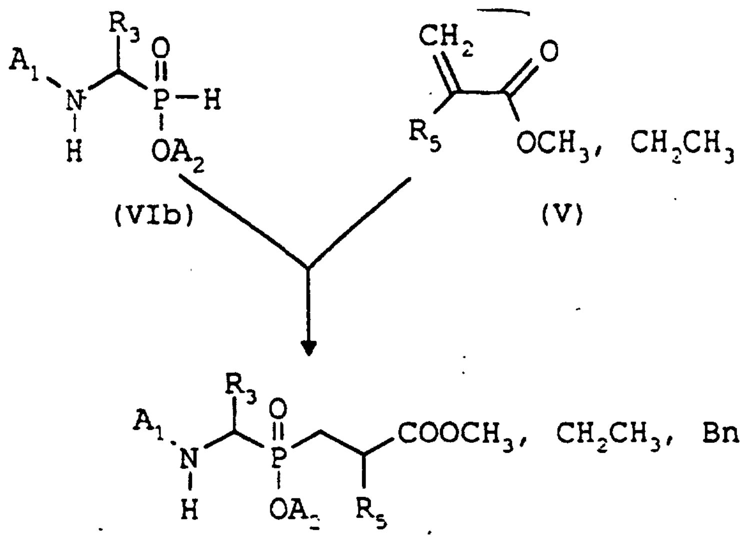

(VIb)

(V)

(IVa)

*schéma 2*

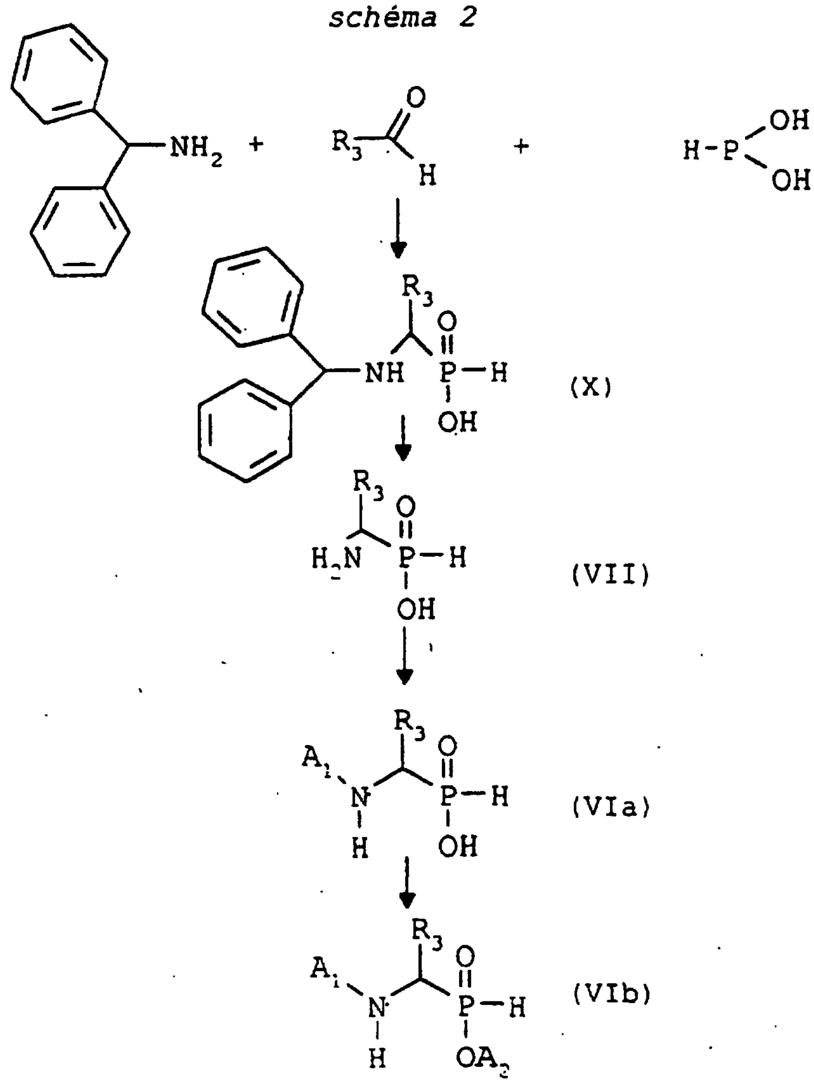

(X)

(VII)

(VIa)

(VIb)

annexe 3

*schéma 3*

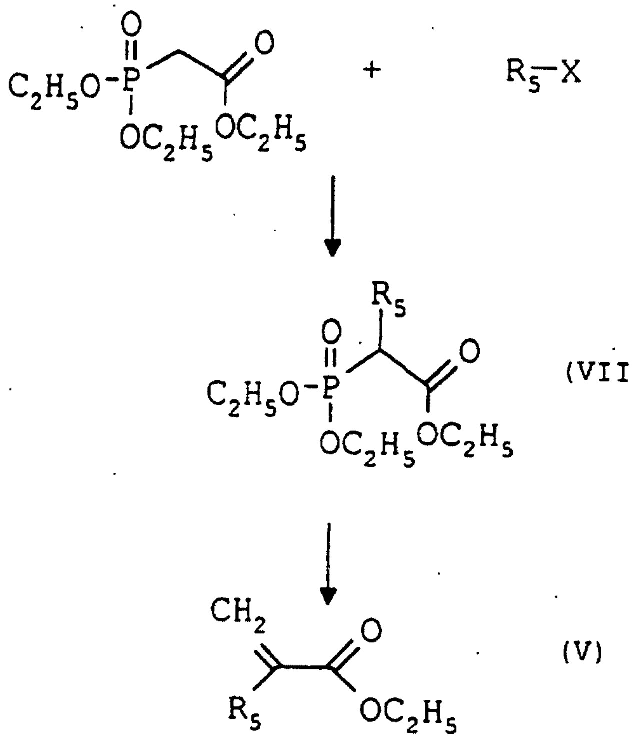

(VIII)

(V)

*schéma 4*

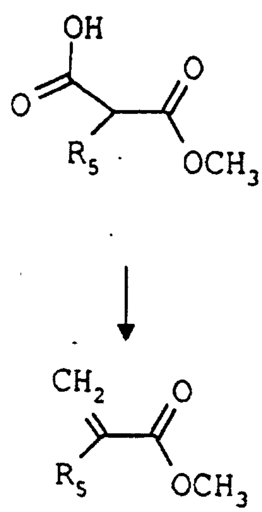

(IX)

(V)

**Revendications**

**1.** Composé de formule (I)

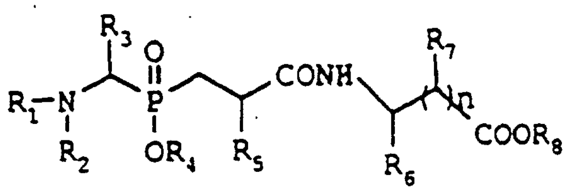

(I)

dans laquelle

- $R_9$ et $R_{10}$ représentent chacun un groupe alkyle de 1 à 6 atomes de carbone,
- $R_1$ et $R_2$ représentent chacun un atome d'hydrogène, ou bien $R_1$ et $R_2$, pris ensemble, forment un groupe insaturé de formule R'(R")C=, dans laquelle,

  - R' représente un groupe phényle monosubstitué en position 2 par un groupe hydroxy ou bien un groupe phényle disubstitué, en position 2, par un groupe hydroxy et, en position 4 ou 5, soit par un atome d'halogène soit par un groupe nitro, soit par un groupe hydroxy, soit par un groupe alcoxy $-OR_9$,
  - R" représente un groupe phényle, un groupe phényle substitué par 1 à 5 atomes d'halogène ou un groupe hétérocyclique aromatique,

  $R_3$ représente

  - un atome d'hydrogène,
  - un groupe alkyle ou un groupe alkényle de 1 à 6 atomes de carbone, ces deux derniers groupes pouvant être substitués par :

    - un groupe hydroxy ou un groupe alcoxy $-OR_9$,
    - un groupe phényle ou un groupe benzyle,
    - un groupe sulfanyle, un groupe alkylsulfanyle $-SR_9$ ou un groupe alkylsulfanyle oxydé sur l'atome de soufre $-S(O)R_9$,
    - un groupe amino, un groupe $-NHR_9$ ou $-NR_9R_{10}$, éventuellement oxydé sur l'atome d'azote ou,
    - un groupe guanidino $H_2N-C(=NH)-NH-$,

  - un groupe cycloalkyle ou cycloalkylméthyle,
  - un groupe phényle, un groupe benzyle, pouvant être substitués sur le groupe phényle par 1 ou 2 des substituants suivants :

    - un atome d'halogène,
    - une groupe hydroxy, un groupe alcoxy $-OR_9$,
    - un groupe alkylsulfanyle $-SR_9$ ou un groupe alkylsulfanyle oxydé sur l'atome de soufre,
    - un groupe amino ou un groupe $-NHR_9$ ou $-NR_9R_10$ éventuellement oxydé sur l'atome d'azote,
    - un groupe nitro,
    - un groupe phényle,
    - un groupe alkyle de 1 à 4 atomes de carbone,

  - un groupe méthyle substitué par un groupe hétérocyclique aromatique ou saturé, les hétéroatomes pouvant être oxydés sous forme de N-oxyde ou de S-oxyde,

  $R_4$ représente

  - un atome d'hydrogène,
  - un groupe alkyle ou alkényle de 1 à 6 atomes de carbone,
  - un groupe cycloalkyle, un groupe cycloalkylalkyle,
  - un groupe phényle, un groupe benzyle, pouvant être substitués sur le groupe phényle par 1 ou 2 des

substituants suivants :

- un groupe alkyle de 1 à 6 atomes de carbone,
- un atome d'halogène,
- un groupe hydroxy ou un groupe alcoxy $-OR_9$,
- un groupe trifluorométhyle,
- un groupe nitro,

$R_5$ représente

- un atome d'hydrogène,
- un groupe alkyle ou un groupe alkényle de 1 à 6 atomes de carbone, ces deux derniers groupes pouvant être substitués par :
  - un groupe hydroxy ou un groupe alcoxy $-OR_9$,
  - un groupe phényle ou un groupe benzyle,
  - un groupe sulfanyle, un groupe alkylsulfanyle $-SR_9$ ou un groupe alkylsulfanyle oxydé sur l'atome de soufre $-S(O)R_9$,
  - un groupe amino, un groupe $-NHR_9$ ou $-NR_9R_{10}$, éventuellement oxydé sur l'atome d'azote ou,
  - un groupe guanidino $H_2N-C(=NH)-NH-$,
- un groupe cycloalkyle ou cycloalkylméthyle,
- un groupe phényle, un groupe benzyle, pouvant être substitués sur le groupe phényle par 1 ou 2 des substituants suivants :
  - un atome d'halogène,
  - une groupe hydroxy, un groupe alcoxy $-OR_9$,
  - un groupe alkylsulfanyle $-SR_9$ ou un groupe alkylsulfanyle oxydé sur l'atome de soufre,
  - un groupe amino ou un groupe $-NHR_9$ ou $-NR_9R_10$ éventuellement oxydé sur l'atome d'azote,
  - un groupe nitro,
  - un groupe phényle,
  - un groupe alkyle de 1 à 4 atomes de carbone,
- un groupe méthyle substitué par un groupe hétérocyclique, les hétéroatomes pouvant être oxydés sous forme de N-oxyde ou de S-oxyde,
- $R_6$ et $R_7$ représentent indépendamment l'un de l'autre
  - un atome d'hydrogène,
  - un groupe alkyle ou alkényle de 1 à 6 atomes de carbone, pouvant être substitué par :
    - > un groupe hydroxy ou un groupe alcoxy $-OR_9$,
    - > un groupe sulfanyle, un groupe alkylsulfanyle $-SR_9$ ou un groupe alkylsulfanyle oxydé sur l'atome de soufre $-S(O)R_9$,
    - > un groupe amino ou un groupe alkylamino $-NHR_9$,
    - > un groupe guanidino $H_2N-C(=NH)-NH-$ ou,
    - > un groupe carboxy ou un groupe alkyloxycarbonyl $-COOR_9$,
  - un groupe phényle, un groupe benzyle, pouvant être substitués sur le groupe phényle par 1 ou 2 substituants suivants :
    - > un atome d'halogène,
    - > un. groupe phényle,
    - > un groupe hydroxy ou un groupe alcoxy $-OR_9$,
    - > un groupe alkylsulfanyle $-SR_9$ ou un groupe alkylsulfanyle oxydé sur l'atome de soufre $-S(O)R_9$,
- $R_6$ et $R_7$ représentent ensemble un cycle saturé ou insaturé à 5 ou 6 chaînons, comprenant 1 ou 2 hétéroatomes pris parmi l'oxygène, le soufre et l'azote,
- $R_8$ représente,

- un atome d'hydrogène,
- un groupe alkyle ou alkényle de 1 à 6 atomes de carbone,
- un groupe phényle, un groupe benzyle,

- n est égal à 0 ou 1,

à l'exception de l'hydrochlorure de *N*-[2-[[(aminométhyl)(méthoxy)phosphinyl]méthyl]-4-méthyl-1-oxopentyl]-(1,1'-biphényl-4-yl)-*L*-alaninate de méthyle.
sous forme d'isomères y compris sous forme d'énantiomères et de diastéréoisomères et de mélanges de ces différentes formes, y compris de mélanges racémiques ainsi que leurs sels d'addition aux acides pharmacologiquement acceptables.

**2.** Composés selon la revendication 1 **caractérisés en ce que** :

- $R_1$, $R_2$, $R_4$, $R_8$ représentent des atomes d'hydrogène,
- n est égal à 0,
- $R_3$ représente

  - un groupe alkyle de 1 à 6 atomes de carbone, pouvant être substitué par un groupe alcoxy $-OR_9$, un groupe sulfanyle ou un groupe alkylsulfanyle $-SR_9$,
  - un groupe phényle, un groupe benzyle, pouvant être substitués sur le groupe phenyle par un atome d'halogène, un groupe alkyle de 1 à 4 atomes de carbone, un groupe alcoxy $-OR_9$ ou un groupe alkylsulfanyle $-SR_9$,
  - $R_5$ représente

    - un groupe alkyle de 1 à 6 atomes de carbone, pouvant être substitué par un groupe alcoxy $-OR_9$, un groupe sulfanyle ou un groupe alkylsulfanyle $-SR_9$,
    - un groupe phényle, un groupe benzyle, pouvant être substitués sur le groupe phényle par un atome d'halogène, un groupe alkyle de 1 à 4 atomes de carbone, un groupe alcoxy $-OR_9$ ou un groupe alkylsulfanyle $-SR_9$,
    - un groupe biphénylméthyle,

  - $R_6$ représente

    - un groupe alkyle de 1 à 6 atomes de carbone, pouvant être substitué par un groupe alcoxy $-OR_9$, un groupe sulfanyle ou un groupe alkylsulfanyle $-SR_9$,
    - un groupe phényle, un groupe benzyle, pouvant être substitués sur le groupe phényle par un atome d'halogène, un groupe alkyle de 1 à 4 atomes de carbone, un groupe alcoxy $-OR_9$ ou un groupe alkylsulfanyle $-SR_9$,
    - un groupe biphénylméthyle,

**3.** Composé de formule (IIa)

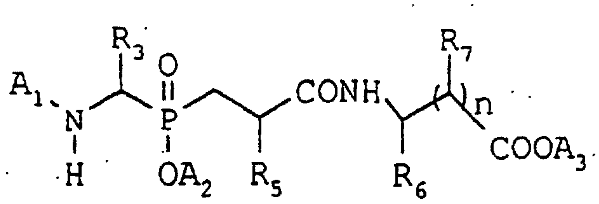
(IIa)

dans laquelle

$A_1$ représente un groupe biphénylméthyle, un groupe tert-butoxycarbonyle, un groupe benzyloxycarbonyle ou un groupe fluorénylméthoxycarbonyle,
$A_2$ représente un atome d'hydrogène, un groupe alkyle ou un groupe benzyle,
$A_3$ représente un groupe méthyle, ethyle, *tert*-butyle ou benzyle,
$R_3$, $R_5$, $R_6$, $R_7$ et n sont tels que définis dans la revendication 1,

à l'exception

du *N*-[2-[[méthoxy[[[(phénylméthoxy)carbonyl]amino]méthyl] phosphinyl]méthyl]-4-méthyl-1-oxopentanyl]-*L*-alaninate de méthyle,
du *N*-[-[[méthoxy[[[(phénylméthoxy)carbonyl]amino]méthyl] phosphinyl]méthyl]-4-méthyl-1-oxopentanyl]-*L*-glycinate de méthyle,
du *N*-[-[[méthoxy[[[(phénylméthoxy)carbonyl]amino]méthyl] phosphinyl]méthyl]-4-méthyl-1-oxopentanyl]-*L*-phénylalaniante de méthyle et,
du *N*-[-[[méthoxy[[[(phénylméthoxy)carbonyl]amino]méthyl] phosphinyl]méthyl]-4-méthyl-1-oxopentanyl]-*L*-leucinate de méthyle,

sous forme d'isomères y compris sous forme d'énantiomères et de diastéréoisomères et de mélanges de ces différentes formes ainsi que sous forme de leurs sels d'addition, utile notamment en tant qu'intermédiaire de synthèse.

**4.** Procédé de préparation des composés de formule (Ia)

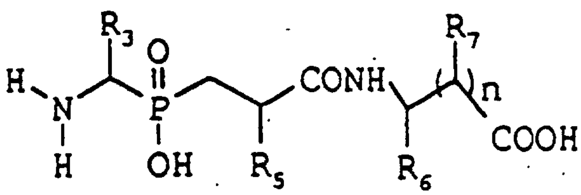

(Ia)

dans laquelle
$R_3$, $R_5$, $R_6$, $R_7$ et n sont tels que définis dans la revendication 1,
qui consiste à :

- traiter un composé de formule (IVb)

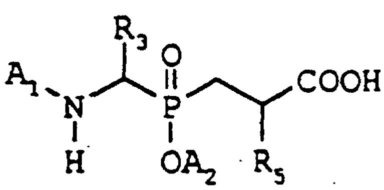

(IVb)

avec un composé de formule (III),

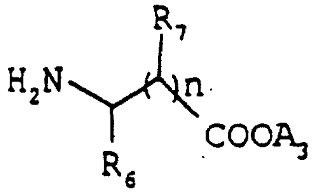

(III)

dans lesquelles
$A_1$, $A_2$, $A_3$ sont tels que définis dans la revendication 3, $R_3$, $R_5$, $R_6$, $R_7$ et n sont tels que définis dans la revendication 1, en présence de benzotriazol-1-yloxytris(dimethylamino)phosphonium hexafluorophosphate pour obtenir un composé de formule (IIa),

- puis à déprotéger les fonctions carboxy, phosphinate et amine simultanément ou successivement pour obtenir un composé de formule (Ia), ce procédé de préparation des composés de formule (Ia) pouvant alternativement consister à déprotéger directement le composé de formule (IIa) par hydrogénation catalytique.

**5.** Procédé de préparation des composés de formule (Ib)

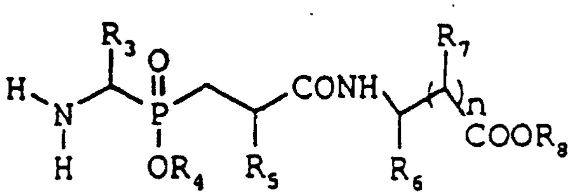

(Ib)

dans laquelle et $R_3$, $R_5$, $R_6$, $R_7$ et n sont tels que définis dans la revendication 1, à condition que $R_4$ et $R_8$ soient différents de l'atome d'hydrogène,
qui consiste :

- à l'estérification du composé de formule (IIc)

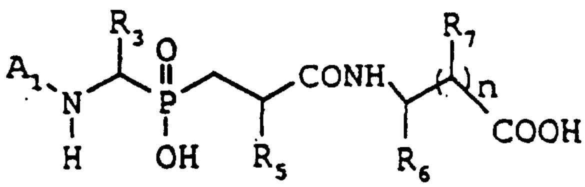

(IIc)

dans lequelle $A_1$ est tel que défini dans La revendication 3 et $R_3$, $R_5$, $R_6$, $R_7$ et n sont tels que définis dans la revendication 1, suivie de la déprotection de la fonction amine ou bien,

- à la déprotection sélective de la fonction aminé du composé de formule (IIa) si $R_4$ et $R_8$ représentent chacun l'un des substituants possibles respectivement de $A_2$, à l'exclusion de l'atome d'hydrogène, et de $A_3$.

**6.** Procédé de préparation des composés de formule (Ic)

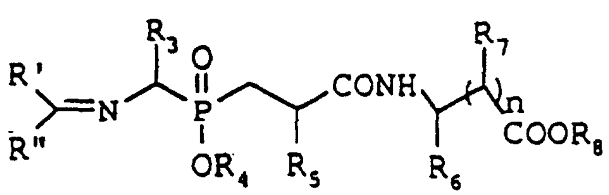

(Ic)

dans laquelle et R', R", $R_3$, $R_4$, $R_5$, $R_6$, $R_7$, $R_8$ et n sont tels que définis dans la revendication 1, qui consiste à condenser une cétone R'(R")C=O sur un composé de formule (Ib) ou (Ia) suivant que les fonctions carboxylates et phosphinates sont protégées ou non, ces cétones R'(R")C=O étant obtenues par réarrangement de Fries des esters correspondants $R"CO_2R'$.

**7.** Médicament **caractérisé en ce qu'**il est constitué par un composé de formule (I) selon la revendication 1 ou 2.

**8.** Composition pharmaceutique **caractérisée en ce qu'**elle contient un composé selon la revendication 1 ou 2, en association avec tout excipient approprié.

## Patentansprüche

**1.** Verbindung der Formel (I)

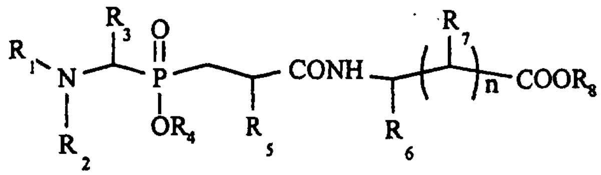

(I)

in welcher

- $R_9$ und $R_{10}$ eine Alkylgruppe mit 1 bis 6 Kohlenstoffatomen darstellen,
- $R_1$ und $R_2$ jeweils ein Wasserstoffatom darstellen oder
- $R_1$ und $R_2$, zusammengenommen, eine ungesättigte Gruppe der Formel R'(R")C= bilden, in welcher

  . R' eine an Position 2 durch ein Hydroxyl monosubstituiertes Phenyl oder ein durch eine Hydroxylgruppe an Position 2 und an Position 4 oder 5, sei es durch ein Halogenatom, sei es durch eine Nitrogruppe, sei es durch eine Hydroxylgruppe, sei es durch eine Alkoxygruppe $-OR_9$ disubstituiertes Phenyl darstellt,
  . R" eine Phenylgruppe, eine durch 1 bis 5 Halogenatome substituierte Phenylgruppe oder eine heterocyclische aromatische Gruppe darstellt,

$R_3$ darstellt

- ein Wasserstoffatom,
- eine Alkylgruppe oder eine Alkenylgruppe mit 1 bis 6 Kohlenstoffatomen, wobei die beiden letzteren Gruppen substituiert sein können durch:

  . eine Hydroxylgruppe oder eine Alkoxygruppe $-OR_9$,
  . eine Phenylgruppe oder eine Benzylgruppe,
  . eine Sulfanylgruppe, eine Alkylsulfanylgruppe $-SR_9$ oder eine Alkylsulfanyloxidgruppe über das Atom des Schwefels $-S(O)R_9$,
  . eine Aminogruppe, eine Gruppe $-NHR_9$ oder $-NR_9R_{10}$, eventuell als Oxid über das Stickstoffatom oder
  . eine Guanidinogruppe $H_2N-C(=NH)-NH-$,

- eine Cycloalkyl- oder Cycloalkylmethylgruppe,
- eine Phenylgruppe, eine Benzylgruppe, die über die Phenylgruppe durch 1 oder 2 der folgenden Substituenten substituiert sein können:

  . ein Halogenatom,
  . eine Hydroxylgruppe, eine Alkoxygruppe $-OR_9$,
  . eine Alkylsulfanylgruppe $-SR_9$ oder eine Alkylsulfanyloxidgruppe über das Atom des Schwefels,
  . eine Aminogruppe oder eine Gruppe $-NHR_9$ oder $-NR_9R_{10}$, eventuell als Oxid über das Stickstoffatom,
  . eine Nitrogruppe,
  . eine Phenylgruppe,
  . eine Alkylgruppe mit 1 bis 4 Kohlenstoffatomen,

- eine Methylgruppe, die durch eine heterocyclische aromatische oder gesättigte Gruppe substituiert ist, wobei die Heteroatome als Oxide in der Form von N-Oxid oder von S-Oxid vorliegen können,

$R_4$ darstellt:

- ein Wasserstoffatom,
- eine Alkyl- oder Alkenylgruppe mit 1 bis 6 Kohlenstoffatomen,
- eine Cycloalkylgruppe, eine Cycloalkylalkylgruppe,
- eine Phenylgruppe, eine Benzylgruppe, wobei die Phenylgruppe mit 1 bis 2 der folgenden Substituenten substituiert sein kann:
  - . eine Alkylgruppe mit 1 bis 6 Kohlenstoffatomen,
  - . ein Halogenatom,
  - . eine Hydroxylgruppe oder eine Alkoxygruppe $-OR_9$,
  - . eine Trifluormethylgruppe,
  - . eine Nitrogruppe,

$R_5$ darstellt:

- ein Wasserstoffatom,
- eine Alkylgruppe oder eine Alkenylgruppe mit 1 bis 6 Kohlenstoffatomen, wobei die beiden letzteren Gruppen substituiert sein können mit:
  - . einer Hydroxylgruppe oder einer Alkoxygruppe $-OR_9$,
  - . einer Phenylgruppe oder einer Benzylgruppe,
  - . einer Sulfanylgruppe, einer Alkylsulfanylgruppe $-SR_9$ oder einer Alkylsulfanylgruppe, die über das Schwefelatom oxidiert ist, $-S(O)R_9$,
  - . einer Aminogruppe, einer Gruppe $-NHR_9$ oder $-NR_9R_{10}$, die eventuell über das Stickstoffatom oxidiert ist, oder
  - . einer Guanidinogruppe $H_2N-C(=NH)-NH-$,
- eine Cykloalkyl- oder Cykloalkylmethylgruppe,
- eine Phenylgruppe, eine Benzylgruppe, die über die Phenylgruppe mit 1 oder 2 der folgenden Substituenten substituiert sein können:
  - . einem Halogenatom,
  - . einer Hydroxylgruppe, einer Alkoxygruppe $-OR_9$,
  - . einer Alkylsulfanylgruppe $-SR_9$ oder einer Alkylsulfanylgruppe, die über das Schwefelatom oxidiert ist,
  - . einer Aminogruppe oder einer Gruppe $-NHR_9$ oder $-NR_9R_{10}$, die eventuell über das Stickstoffatom oxidiert ist,
  - . einer Nitrogruppe,
  - . einer Phenylgruppe,
  - . einer Alkylgruppe mit 1 bis 4 Kohlenstoffatomen,
- eine Methylgruppe, die durch eine heterocyclische Gruppe substituiert ist, wobei die Heteroatome in der Form von N-Oxid oder von S-Oxid oxidiert sein können,

$R_6$ und $R_7$ unabhängig voneinander darstellen:

- ein Wasserstoffatom,
- eine Alkyl- oder Alkenylgruppe mit 1 bis 6 Kohlenstoffatomen, die substituiert sein können durch:
  - . eine Hydroxyl- oder eine Alkoxygruppe $-OR_9$,
  - . eine Sulfanylgruppe, eine Alkylsulfanylgruppe $-SR_9$ oder eine Alkylsulfanylgruppe, die über das Schwefelatom oxidiert ist, $-S(O)R_9$,
  - . eine Aminogruppe oder eine Alkylaminogruppe-$NHR_9$,
  - . eine Guanidinogruppe $H_2N-C(=NH)-NH-$ oder
  - . eine Carboxygruppe oder eine Alkyloxycarbonylgruppe $-COOR_9$,
- eine Phenylgruppe, eine Benzylgruppe, die über die Phenylgruppe durch 1 oder 2 der folgenden Substituenten substituiert sein können:

- ein Halogenatom,
- eine Phenylgruppe,
- eine Hydroxylgruppe oder eine Alkoxygruppe $-OR_9$,
- eine Alkylsulfanylgruppe $-SR_9$ oder eine Alkylsulfanylgruppe, die über das Schwefelatom oxidiert ist, $-S(O)R_9$,

• $R_6$ und $R_7$ zusammen einen gesättigten Zyklus oder einen ungesättigten Zyklus mit 5 oder 6 Kettengliedern darstellen, die 1 oder 2 Heteroatome, ausgewählt unter Sauerstoff, Schwefel und Stickstoff umfassen,

• $R_8$ darstellt:

- ein Wasserstoffatom,
- eine Alkyl- oder Alkenylgruppe mit 1 bis 6 Kohlenstoffatomen,
- eine Phenylgruppe, eine Benzylgruppe,

• n entspricht 0 oder 1,

ausgenommen von dem Hydrochlorid von N-[2-[[(aminomethyl)(methoxy) phosphinyl]methyl]-4-methyl-1-oxopentyl]-(1,1'-biphenyl-4-yl)-L-alaninat von Methyl,

in der Form von Isomeren, eingeschlossen in der Form von Enantiomeren und von Diasteromeren und von Mischungen von diesen verschiedenen Formen, wobei umfasst sind Racemat-Mischungen, genauso wie deren Additionssalze mit pharmakologisch akzeptablen Säuren.

**2.** Verbindungen gemäß Anspruch 1, **dadurch gekennzeichnet, dass**:

• $R_1$, $R_2$, $R_4$, $R_8$ Wasserstoffatome darstellen,
• n entspricht 0,
• $R_3$ darstellt:

- eine Alkylgruppe mit 1 bis 6 Kohlenstoffatomen, die durch eine Alkoxygruppe $-OR_9$, eine Sulfanylgruppe oder eine Alkylsulfanylgruppe $-SR_9$ substituiert sein kann,
- eine Phenylgruppe, eine Benzylgruppe, die über die Phenylgruppe durch ein Halogenatom, eine Alkylgruppe mit 1 bis 4 Kohlenstoffatomen, eine Alkoxygruppe $-OR_9$ oder eine Alkylsulfanylgruppe $-SR_9$ substituiert sein können,

• $R_5$ darstellt:

- eine Alkylgruppe mit 1 bis 6 Kohlenstoffatomen, die durch eine Alkoxygruppe $-OR_9$, eine Sulfanylgruppe oder eine Alkylsulfanylgruppe $-SR_9$ substituiert sein können,
- eine Phenylgruppe, eine Benzylgruppe, die über die Phenylgruppe durch ein Halogenatom, eine Alkylgruppe mit 1 bis 4 Kohlenstoffatomen, eine Alkoxygruppe $-OR_9$ oder eine Alkylsulfanylgruppe $-SR_9$ substituiert sein können,
- eine Biphenylmethylgruppe,

• $R_6$ darstellt:

- eine Alkylgruppe mit 1 bis 6 Kohlenstoffatomen, die durch eine Alkoxygruppe $-OR_9$, eine Sulfanylgruppe oder eine Alkylsulfanylgruppe $-SR_9$ substituiert sein kann,
- eine Phenylgruppe, eine Benzylgruppe, die über die Phenylgruppe substituiert sein können durch ein Halogenatom, eine Alkylgruppe mit 1 bis 4 Kohlenstoffatomen, eine Alkoxygruppe $-OR_9$ oder eine Alkylsulfanylgruppe $-SR_9$,
- eine Biphenylmethylgruppe,

**3.** Verbindung der Formel (IIa)

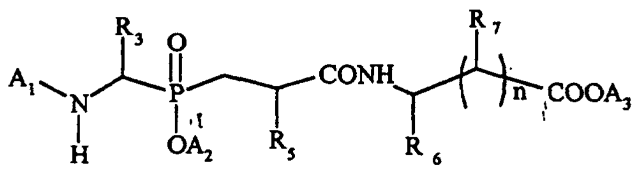

(IIa)

in welcher

$A_1$ darstellt: eine Biphenylmethylgruppe, eine tert-Butoxycarbonylgruppe, eine Benzyloxycarbonylgruppe oder eine Fluorenylmethoxycarbonylgruppe,
$A_2$ darstellt: ein Hydrogenatom, eine Alkylgruppe oder eine Benzylgruppe,
$A_3$ darstellt: eine Methyl-, Ethyl-, tert-Butyl- oder Benzylgruppe,

wobei $R_3$, $R_5$, $R_6$, $R_7$ und n wie in Anspruch 1 definiert sind,
mit Ausnahme von:

N-[2-[[methoxy[[[(phenylmethoxy)carbonyl]amino]-methyl]phosphinyl]methyl]-4-methyl-1-oxopentanyl]-L-alaninat von Methyl;
N-[-[[methoxy[[[(phenylmethoxy)carbonyl]amino]-methyl]phosphinyl]methyl]-4-methyl-1-oxopentanyl]-L-glycinat von Methyl;
N-[-[[methoxy[[[(phenylmethoxy)carbonyl]amino]-methyl]phosphinyl]methyl]-4-methyl-1-oxopentanyl]-L-phenylalaninat von Methyl, und
N-[-[[methoxy[[[(phenylmethoxy)carbonyl]amino]-methyl]phosphinyl]methyl]-4-methyl-1-oxopentanyl]-L-Leucinat von Methyl

in der Form von Isomeren, eingeschlossen in der Form von Enantiomeren und von Diastereoisomeren und von Mischungen von diesen verschiedenen Formen, genauso wie in Form von deren Additionssalzen, die insbesondere als Synthese-Zwischenstufe nützlich sind.

**4.** Verfahren zur Herstellung von Verbindungen der Formel (Ia)

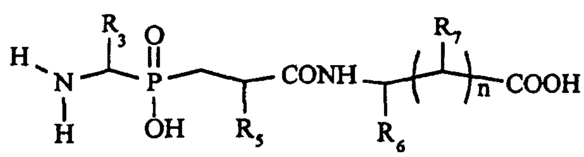

(Ia)

in welcher
$R_3$, $R_5$, $R_6$, $R_7$ und n so wie in Anspruch 1 definiert sind,
welches umfasst:

- Behandeln einer Verbindung der Formel (IVb)

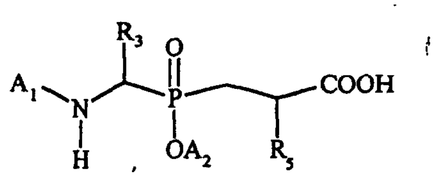

(IVb)

mit einer Verbindung der Formel (III),

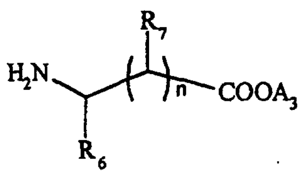

(III)

in welchen
$A_1$, $A_2$, $A_3$ wie in Anspruch 3 definiert sind,
$R_3$, $R_5$, $R_6$ und $R_7$ und n wie in Anspruch 1 definiert sind, in Gegenwart von Benzotriazol-1-yloxy-tris(dimethylamino)phosphoniumhexafluorphosphat, um eine Verbindung der Formel (IIa) zu erhalten,

- dann Schutzgruppen-Entfemen von den Funktionen Carboxy, Phosphinat und Amin gleichzeitig oder aufeinander folgend, um eine Verbindung der Formel (Ia) zu erhalten,

wobei das Verfahren der Herstellung von Verbindungen der Formel (Ia) alternativ aus direkter Schutzgruppen-Entfernung von der Verbindung der Formel (IIa) durch katalytische Hydrierung bestehen kann.

**5.** Verfahren zur Herstellung von Verbindungen der Formel (Ib)

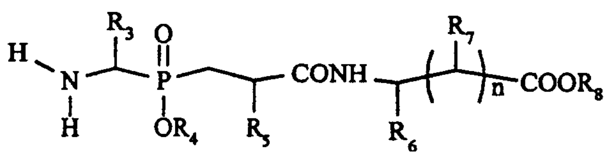

(Ib)

in welcher $R_3$, $R_5$, $R_6$, $R_7$ und n wie in Anspruch 1 definiert sind, unter der Bedingung, dass $R_4$ und $R_8$ sich von dem Wasserstoffatom unterscheiden, welches umfasst:

- eine Veresterung der Verbindung der Formel (IIc)

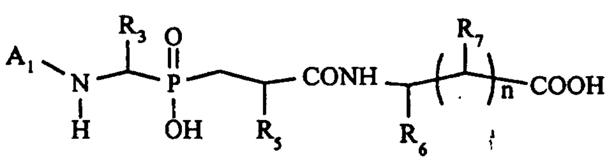

(IIc)

in welcher $A_1$ so wie in Anspruch 3 definiert ist und $R_3$, $R_5$, $R_6$, $R_7$ und n so wie in Anspruch 1 definiert sind, gefolgt von der Schutzgruppen-Entfernung von der Aminfunktion oder

- selektive Schutzgruppen-Entfernung von der Aminfunktion von der Verbindung der Formel (IIa), wenn $R_4$ und $R_8$ jeweils eine der möglichen Substituenten jeweils von $A_2$, mit Ausschluss von dem Wasserstoffatom, und von $A_3$ darstellen.

**6.** Verfahren zur Herstellung von Verbindungen der Formel (Ic)

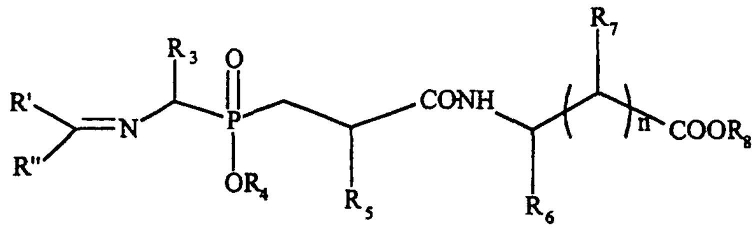

(Ic)

in welcher R', R", $R_3$, $R_4$, $R_5$, $R_6$, $R_7$, $R_8$ und n wie in Anspruch 1 definiert sind, welches umfasst: Kondensieren eines Ketons R'(R")C=O mit einer Verbindung der Formel (Ib) oder (Ia), gefolgt von dem Schützen der Carboxylat- und Phosphinat-Funktionen oder nicht, wobei die Ketone R'(R")C=O durch Fries-Umlagerung von den entsprechenden Estern R"$CO_2$R', erhalten werden.

7. Medikament, **dadurch gekennzeichnet, dass** dieses eine Verbindung der Formel (I) gemäß Anspruch 1 oder 2 umfasst.

8. Pharmazeutische Zusammensetzung, **dadurch gekennzeichnet, dass** diese eine Verbindung gemäß Anspruch 1 oder 2, zusammen mit einem geeigneten Träger umfasst.

## Claims

1. Compound of formula (I)

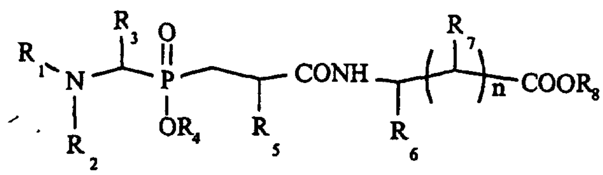

(I)

in which

- $R_9$ and $R_{10}$ represent an alkyl group of 1 to 6 carbon atoms,
- $R_1$ and $R_2$ each represent a hydrogen atom or alternatively $R_1$ and $R_2$, taken together form an unsaturated group of formula R'(R")C=, in which,

  . R' represents a phenyl group monosubstituted in position 2 with a hydroxyl group, or alternatively a phenyl group disubstituted, in position 2, with a hydroxyl group and, in position 4 or 5, either with a halogen atom or with a nitro group, or with a hydroxyl group, or with an alkoxy group $-OR_9$,
  . R" represents a phenyl group, a phenyl group substituted by 1 to 5 halogen atoms or a heterocyclic aromatic group,

  $R_3$ represents

  - a hydrogen atom,
  - an alkyl group or an alkenyl group of 1 to 6 carbon atoms, it being possible for these last two groups to be substituted with:

    . a hydroxyl group ao an alkoxy group $-OR_9$,
    . a phenyl group or benzyl group,
    . a sulphanyl group, an alkylsulphanyl group $-SR_9$ or an alkylsulphanyl group oxidized on the sulphur atom $-S(O)R_9$,
    . an amino group, a group $-NHR_9$ or $-NR_9R_{10}$, optionally oxidized on the nitrogen atom, or
    . a guanidino group $H_2N-C(=NH)-NH-$,

- a cycloalkyl or cycloalkylmethyl group,
- a phenyl group, a benzyl group, which can be substituted on the phenyl group with 1 or 2 of the following substituents:
  - a halogen atom,
  - a hydroxyl group, an alkoxy group $-OR_9$,
  - an alkylsulphanyl group $-SR_9$ or an alkylsulphanyl group oxidized on the sulphur atom,
  - an amino group or a group $-NHR_9$ or $-NR_9R_{10}$ optionally oxidized on the nitrogen atom,
  - a nitro group,
  - a phenyl group,
  - an alkyl group of 1 to 4 carbon atoms,
- a methyl group substituted with a heterocyclic aromatic or saturated group, it being possible for the hetero atoms to be oxidized in the form of N-oxide or S-oxide,

$R_4$ represents

- a hydrogen atom,
- an alkyl or alkenyl group of 1 to 6 carbon atoms,
- a cycloalkyl group, a cycloalkylalkyl group,
- a phenyl group, a benzyl group, which can be substituted on the phenyl group with 1 or 2 of the following substituents:
  - an alkyl group of 1 to 6 carbon atoms,
  - a halogen atom,
  - a hydroxyl group or an alkoxy group $-OR_9$,
  - a trifluoromethyl group,
  - a nitro group,

$R_5$ represents

- a hydrogen atom,
- an alkyl group or an alkenyl group of 1 to 6 carbon atoms, it being possible for these last two groups to be substituted with:
  - a hydroxyl group or an alkoxy group $-OR_9$,
  - a phenyl group or a benzyl group,
  - a sulphanyl group, an alkylsulphanyl group $-SR_9$, or an alkylsulphanyl group oxidized on the sulphur atom $-S(O)R_9$,
  - an amino group, a group $-NHR_9$ or $-NR_9R_{10}$, optionally oxidized on the nitrogen atom, or
  - a guanidine group $H_2N-C(=NH)-NH-$,
- a cycloalkyl or cycloalkylmethyl group,
- a phenyl group, a benzyl group, which can be substituted on the phenyl group with 1 or 2 of the following substituents:
  - a halogen atom,
  - a hydroxyl group, an alkoxy group $-OR_9$,
  - an alkylsulphanyl group $-SR_9$, or an alkylsulphanyl group oxidized on the sulphur atom,
  - an amino group or a group $-NHR_9$ or $-NR_9R_{10}$ optionally oxidized on the nitrogen atom,
  - a nitro group,
  - a phenyl group,
  - an alkyl group of 1 to 4 carbon atoms,
- a methyl group substituted with a heterocyclic group, it being possible for the hetero atoms to be oxidized in the form of N-oxide or S-oxide,

$R_6$ and $R_7$ represent, independently of each other,

- a hydrogen atom,
- an alkyl or alkenyl group of 1 to 6 carbon atoms, which can be substituted with:
  - a hydroxyl group or an alkoxy group $-OR_9$,
  - a sulphanyl group, an alkylsulphanyl group $-SR_9$ or an alkylsulphanyl group oxidized on the sulphur atom $-S(O)R_9$,
  - an amino group or an alkylamino group $-NHR_9$,
  - a guanidine group $H_2N-C(=NH)-NH-$, or
  - a carboxyl group or an alkyloxycarbonyl group $-COOR_9$,
- a phenyl group, a benzyl group, which can be substituted on the phenyl group by 1 or 2 of the following substituents:
  - a halogen atom,
  - a phenyl group,
  - a hydroxyl group or an alkoxy group $-OR_9$,
  - an alkylsulphanyl group $-SR_9$, or an alkylsulphanyl group oxidized on the sulphur atom $-S(O)R_9$,
- $R_6$ and $R_7$ together represent a saturated or unsaturated 5- or 6-membered ring comprising 1 or 2 hetero atoms, taken from among oxygen, sulphur and nitrogen,
- $R_8$ represents,
  - a hydrogen atom,
  - an alkyl or alkenyl group of 1 to 6 carbon atoms,
  - a phenyl group, a benzyl group,
- n is equal to 0 or 1,

with the exception of methyl N-[2-[[(aminomethyl)(methoxy)phosphinyl]methyl]-4-methyl-1-oxopentyl]-(1,1'-biphenyl-4-yl)-L-alaninate hydrochloride,

in the form of isomers, including the form of enantiomers and diastereoisomers and mixtures of these different forms, including racemic mixtures, as well as the addition salts thereof with pharmacologically acceptable acids.

**2.** Compounds according to claim 1, **characterized in that**:

- $R_1$, $R_2$, $R_4$ and $R_8$ represent hydrogen atoms,
- n is equal to 0,
- $R_3$ represents
  - an alkyl group of 1 to 6 carbon atoms, which can be substituted with an alkoxy group $-OR_9$, a sulphanyl group or an alkylsulphanyl group $-SR_9$,
  - a phenyl group, a benzyl group, which can be substituted on the phenyl group with a halogen atom, an alkyl group of 1 to 4 carbon atoms, an alkoxy group $-OR_9$ or an alkylsulphanyl group $-SR_9$,
- $R_5$ represents
  - an alkyl group of 1 to 6 carbon atoms, which can be substituted with an alkoxy group $-OR_9$, a sulphanyl group or an alkylsulphanyl group $-SR_9$,
  - a phenyl group, a benzyl group, which can be substituted on the phenyl group with a halogen atom, an alkyl group of 1 to 4 carbon atoms, an alkoxy group $-OR_9$ or an alkylsulphanyl group $-SR_9$,
  - a biphenylmethyl group,
- $R_6$ represents
  - an alkyl group of 1 to 6 carbon atoms, which can be substituted with an alkoxy group $-OR_9$, a sulphanyl group or an alkylsulphanyl group $-SR_9$,
  - a phenyl group, a benzyl group, which can be substituted on the phenyl group with a halogen atom, an alkyl group of 1 to 4 carbon atoms, an alkoxy group $-OR_9$ or an alkylsulphanyl group $-SR_9$,

. a biphenylmethyl group.

**3.** Compound of formula (IIa)

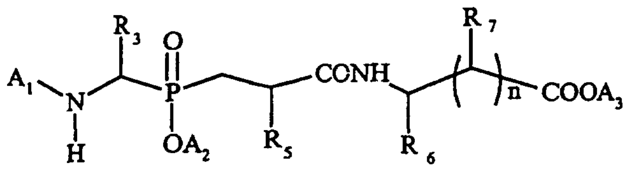

**(IIa)**

in which

$A_1$ represents a biphenylmethyl group, a tert-butoxy-carbonyl group, a benzyloxycarbonyl group or a fluorenylmethoxycarbonyl group,
$A_2$ represents a hydrogen atom, an alkyl group or a benzyl group,
$A_3$ represents a methyl, ethyl, tert-butyl or benzyl group,
$R_3$, $R_5$, $R_6$, $R_7$ and n are as defined in claim 1, with the exception
of methyl N-[2-[[methoxy[[[(phenylmethoxy)carbonyl]amino]methyl]phosphinyl] methyl]-4-methyl-1-oxopentanyl]-L-alaninate,
of methyl N-[-[[methoxy[[[(phenylmethoxy)carbonyl]amino]methyl]phosphinyl] methyl]-4-methyl-1-oxopentanyl]-L-glycinate,
of methyl N-[-[[methoxy[[[(phenylmethoxy)carbonyl]amino]methyl]phosphinyl] methyl]-4-methyl-1-oxopentanyl]-L-phenylalaninate and,
of methyl N-[-[[methoxy[[[(phenylmethoxy)carbonyl]amino]methyl]phosphinyl] methyl]-4- methyl-1-oxopentanyl]-L-leucinate,

in the form of isomers, including in the form of the enantiomers and diastereoisomers and mixtures of these different forms, as well as the addition salts thereof, that are useful particularly as synthesis intermediates.,

**4.** Process for the preparation of the compounds of formula (Ia)

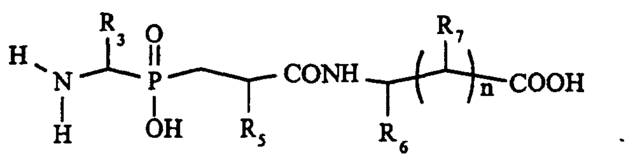

**(Ia)**

in which $R_3$, $R_5$, $R_6$, $R_7$ and n are as defined in claim 1, which comprises:

- treating a compound of formula (IVb)

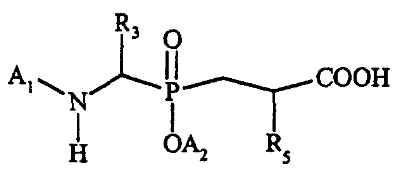

**(IVb)**

with a compound of formula (III) in which

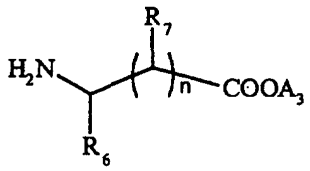

(III)

$A_1$, $A_2$, $A_3$ are as defined in claim 3,
$R_3$, $R_5$, $R_6$, $R_7$ and n are as defined in claim 1, in the presence of benzotriazol-1-yloxytris(dimetylamino)phosphonium hexafluorophosphate, in order to obtain a compound of formula (IIa),

- and then in deprotecting the carboxyl, phosphinate and amine functions simultaneously or successively, in order to obtain a compound of formula (Ia),

it being alternatively possible for this process for the preparation of the compounds of formula (Ia) to consist in deprotecting the compound of formula (IIa) directly by catalytic hydrogenation.

**5.** Process for the preparation of the compounds of formula (Ib)

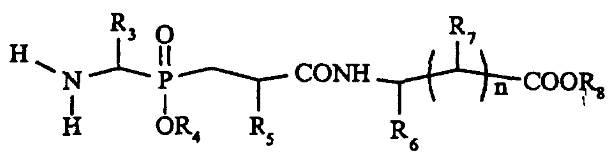

(Ib)

in which $R_3$, $R_5$, $R_6$, $R_7$ and n are as defined in claim 1, with the proviso that $R_4$ and $R_8$ are other than a hydrogen atom,
which comprises:

- esterification of the compound of formula (IIc)

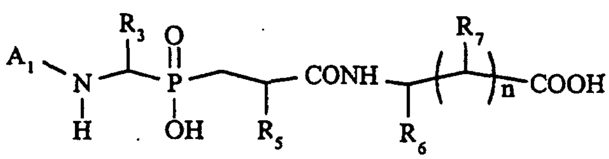

(IIc)

in which $A_1$ is as defined in claim 3, and $R_3$, $R_5$, $R_6$, $R_7$ and n are as defined in claim 1, followed by deprotection of the amine function, or alternatively

- selective deprotection of the amine function in this compound of formula (IIa) if $R_4$ and $R_8$ each represent one of the possible substituants, respectively of $A_2$, with the exclusion of a hydrogen atom, and of $A_3$.

**6.** Process for the preparation of the compounds of formula (Ic)

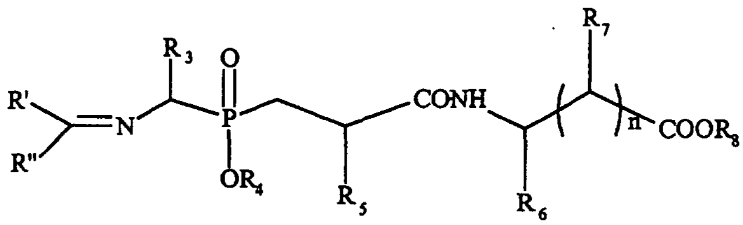

(Ic)

in which R', R", $R_3$, $R_4$, $R_5$, $R_6$, $R_7$, $R_8$ and n are as defined in claim 1, which consists in condensing a ketone R' (R")C=O with a compound of formula (Ib) or (Ia), depending on whether or not the carboxylate and phosphinate functions are protected, these ketones R'(R")C=O being obtained by Fries rearrangement of the corresponding esters $R''CO_2R'$.

**7.** Drug, **characterized in that** it comprises a compound of formula (I) according to claim 1 or 2.

**8.** Pharmaceutical composition, **characterized in that** it contains a compound according to claim 1 or 2, in combination with any suitable excipient.